# EUROPÄISCHE PATENTANMELDUNG

(11) **EP 1 702 518 A1**
(43) Veröffentlichungstag der Anmeldung: **20.09.2006**
(21) Anmeldenummer: 05024607.3
(22) Anmeldetag: 19.09.1996
(51) Int. Cl.: A23L 1/0522, C12N 15/82

(54) **Pflanzen, die eine modifizierte Stärke synthetisieren, Verfahren zu ihrer Herstellung sowie modifizierte Stärke**

(30) Priorität: 19.09.1995 DE 19534759; 20.12.1995 DE 19547733
(62) Teilanmeldung aus: 96932575.2
(71) Anmelder: Bayer BioScience GmbH, 14473 Potsdam (DE)
(72) Erfinder: Kossmann, Jens, Somerset West 7130 (ZA); Lorberth, Ruth, San Francisco, CA 94123 (US)
(74) Vertreter: Vossius & Partner

(57) **Zusammenfassung**

Es werden Nucleinsäuremoleküle beschrieben, die ein Stärkekorn-gebundenes Protein codieren, sowie Verfahren und rekombinante DNA-Moleküle zur Herstellung transgener Pflanzenzellen und Pflanzen, die eine modifizierte Stärke mit veränderten Viskositätseigenschaften und einem veränderten Phosphatgehalt synthetisieren. Darüberhinaus werden die aus den Verfahren resultierenden Pflanzenzellen und Pflanzen und die aus ihnen erhältliche Stärke beschrieben.

## Beschreibung

Die vorliegende Erfindung betrifft Nucleinsäuremoleküle, die ein Stärkekorn-gebundenes Protein codieren, sowie Verfahren und rekombinante DNA-Moleküle zur Herstellung transgener Pflanzenzellen und Pflanzen, die eine modifizierte Stärke mit veränderten Viskositätseigenschaften und einem veränderten Phosphatgehalt synthetisieren. Die Erfindung betrifft ebenfalls die aus den Verfahren resultierenden transgenen Pflanzenzellen und Pflanzen und die aus den transgenen Pflanzenzellen und Pflanzen erhältliche Stärke.

Das Polysaccharid Stärke, das einen der wichtigsten Speicherstoffe im Pflanzenreich darstellt, findet neben der Verwendung im Nahrungsmittelbereich auch eine breite Verwendung als nachwachsender Rohstoff für die Herstellung industrieller Produkte. Um die Anwendung dieses Rohstoffes in möglichst vielen Einsatzgebieten zu ermöglichen, ist es notwendig, eine große Stoffvielfalt und eine Anpassung an die jeweiligen Anforderungen der zu verarbeitenden Industrie zu erreichen.
Obwohl Stärke aus einem chemisch einheitlichen Grundbaustein, der Glucose, aufgebaut ist, stellt Stärke keinen einheitlichen Rohstoff dar. Es handelt sich dabei eher um ein komplexes Gemisch aus unterschiedlichen Molekülformen, die sich hinsichtlich ihres Verzweigungsgrades und des Auftretens von Verzweigungen der Glucoseketten unterscheiden. Man unterscheidet insbesondere die Amylose-Stärke, ein im wesentlichen unverzweigtes Polymer aus α-1,4-verknüpften Glucosemolekülen, von der Amylopektin-Stärke, die ein Gemisch aus unterschiedlich stark verzweigten Glucoseketten darstellt, wobei die Verzweigungen durch das Auftreten von α-1,6-glycosidischen Verknüpfungen zustande kommen.

Die molekulare Struktur der Stärke, die zu einem großen Teil durch den Verzweigungsgrad, das Amylose/AmylopektinVerhältnis, die durchschnittliche Kettenlänge sowie das Vorhandensein von Phosphatgruppen bestimmt wird, ist ausschlaggebend für wichtige funktionelle Eigenschaften der Stärke bzw. ihrer wäßrigen Lösungen. Als wichtige funktionelle Eigenschaften sind hierbei beispielsweise zu nennen die Löslichkeit, das Retrogradierungsverhalten, die Filmbildungseigenschaften, die Viskosität, die Farbstabilität, die Verkleisterungseigenschaften, d.h. Binde- und Klebeigenschaften, sowie die Kältestabilität. Auch die Stärkekorngröße kann für verschiedene Anwendungen von Bedeutung sein. Von besonderem Interesse ist insbesondere die Erzeugung von hochamylosehaltigen Stärken. Ferner kann eine in Pflanzenzellen enthaltene modifizierte Stärke das Verhalten der Pflanzenzelle unter bestimmten Bedingungen vorteilhaft verändern. Denkbar ist beispielsweise eine Verringerung des Stärkeabbaus während der Lagerung von Stärke-enthaltenden Organen, wie z.B. Samen oder Knollen, vor deren weiterer Verarbeitung, z.B. zur Extraktion der Stärke. Ferner ist es von Interesse, modifizierte Stärken herzustellen, die dazu führen, daß Pflanzenzellen oder pflanzliche Organe, die diese Stärke enthalten, besser zur Weiterverarbeitung geeignet sind, beispielsweise bei der Herstellung von "Popcorn" oder "Corn flakes" aus Mais oder von Pommes frites, Chips oder Kartoffelpulver aus Kartoffeln. Von besonderem Interesse ist hierbei die Verbesserung der Stärken in der Hinsicht, daß sie ein reduziertes "cold sweetening" aufweisen, d.h. eine verringerte Freisetzung von reduzierenden Zuckern (insbesondere Glucose) bei einer längeren Lagerung bei niedrigen Temperaturen. Gerade Kartoffeln werden häufig bei Temperaturen von 4-8 °C gelagert, um den Stärkeabbau während der Lagerung zu minimieren. Die hierbei freigesetzten reduzierenden Zucker, insbesondere Glucose, führen beispielsweise bei der Herstellung von Pommes frites oder Chips zu unerwünschten Bräunungsreaktionen (sogenannte Maillard-Reaktionen).
Die Anpassung der aus Pflanzen isolierbaren Stärke an bestimmte industrielle Verwendungszwecke erfolgt häufig mit Hilfe chemischer Modifikationen, die in der Regel zeit- und kostenintensiv sind. Es erscheint daher wünschenswert, Möglichkeiten zu finden, Pflanzen herzustellen, die eine Stärke synthetisieren, die in ihren Eigenschaften bereits den Anforderungen der verarbeitenden Industrie entspricht.
Herkömmliche Wege zur Herstellung derartiger Pflanzen bestehen in klassischen Züchtungsverfahren und der Erzeugung von Mutanten. So wurde beispielsweise bei Mais eine Mutante erzeugt, die eine Stärke mit veränderten Viskositätseigenschaften synthetisiert (US Patentschrift 5,331,108), sowie eine Maissorte (waxy *maize*) durch Züchtung etabliert, deren Stärke zu nahezu 100% aus Amylopektin besteht (Akasuka und Nelson, J. Biol. Chem. 241 (1966), 2280-2285). Ferner sind bei Mais und Erbse Mutanten beschrieben worden, die Stärken mit hohem Amylosegehalt synthetisieren (70% in Mais bzw. bis zu 50% in Erbse). Diese Mutanten sind bisher nicht auf molekularer Ebene charakterisiert worden und erlauben somit auch nicht die Erzeugung entsprechender Mutanten in anderen stärkespeichernden Pflanzen.
Alternativ können Pflanzen, die eine Stärke mit veränderten Eigenschaften synthetisieren, mit Hilfe gentechnischer Verfahren erzeugt werden. Beschrieben wurde beispielsweise in mehreren Fällen die gentechnische Veränderung von Kartoffelpflanzen, mit dem Ziel der Veränderung der in den Pflanzen synthetisierten Stärke (z.B. WO 92/11376; WO 92/14827). Voraussetzung für die Anwendung gentechnischer Verfahren ist jedoch die Verfügbarkeit von DNA-Sequenzen, deren Genprodukte einen Einfluß auf die Stärkesynthese, die Stärkemodifikation oder den Stärkeabbau haben.

Der vorliegenden Erfindung liegt daher die Aufgabe zugrunde, Nucleinsäuremoleküle und Verfahren zur Verfügung zu stellen, die es ermöglichen, Pflanzen dahingehend zu verändern, daß sie eine Stärke synthetisieren, die sich hinsichtlich ihrer physikalischen und/oder chemischen Eigenschaften von natürlicherweise in den Pflanzen synthetisierter Stärke unterscheidet, insbesondere eine hochamylosehaltige Stärke, und die somit für allgemeine und/oder spezielle Verwendungszwekke besser geeignet ist.

Diese Aufgabe wird durch die Bereitstellung der in den Patentansprüchen bezeichneten Ausführungsformen gelöst.

Die vorliegende Erfindung betrifft somit Nucleinsäuremoleküle, die ein Protein mit der unter Seq ID No. 2 angegebenen Aminosäuresequenz codieren. Derartige Proteine liegen in den Plastiden pflanzlicher Zellen sowohl an Stärkekörnern gebunden vor, als auch außerhalb von Stärkekörnern in freier, d.h. löslicher Form. Die Enzymaktivität derartiger Proteine führt bei Expression in E. coli zu einer erhöhten Phosphorylierung des in den Zellen synthetisierten Glycogens. Das Molekulargewicht dieser Proteine liegt im Bereich von 140-160 kd, wenn es mit Hilfe einer SDS-Gelelektrophorese bestimmt wird.

Ferner betrifft die vorliegende Erfindung Nucleinsäuremoleküle, die eine Sequenz mit der unter Seq ID No. 1 angegebenen Nucleotidabfolge, insbesondere die in Seq ID No. 1 angegebenen codierenden Region, umfassen.

Gegenstand der Erfindung sind ebenfalls Nucleinsäuremoleküle, die ein Protein codieren, das in den Plastiden pflanzlicher Zellen zum Teil an Stärkekörnern gebunden vorliegt, und die mit den oben genannten erfindungsgemäßen Nucleinsäuremolekülen hybridisieren. Der Begriff "Hybridisierung" bedeutet in diesem Zusammenhang eine Hybridisierung unter konventionellen Hybridisierungsbedingungen, vorzugsweise unter stringenten Bedingungen, wie sie beispielsweise in Sambrook et al. (1989, Molecular Cloning, A Laboratory Manual, 2. Aufl. Cold Spring Harbor Laboratory Press, Cold Spring Harbor, NY) beschrieben sind. Diese Nucleinsäuremoleküle, die mit den erfindungsgemäßen Nucleinsäuremolekülen hybridisieren, können prinzipiell aus jedem beliebigen Organismus (d.h. Prokaryonten oder Eukaryonten, insbesondere aus Bakterien, Pilzen, Algen, Pflanzen oder tierischen Organismen) stammen, der derartige Nucleinsäuremoleküle besitzt. Sie stammen vorzugsweise aus monokotylen oder dikotylen Pflanzen, insbesondere aus Nutzpflanzen, und besonders bevorzugt aus Stärkespeichernden Pflanzen.
Nucleinsäuremoleküle, die mit den erfindungsgemäßen Molekülen hybridisieren, können z.B. aus genomischen oder aus cDNA-Bibliotheken verschiedener Organismen isoliert werden.
Die Identifizierung und Isolierung derartiger Nucleinsäuremoleküle aus Pflanzen oder anderen Organismen kann dabei unter Verwendung der erfindungsgemäßen Nucleinsäuremoleküle oder Teile dieser Moleküle bzw. der reversen Komplemente dieser Moleküle erfolgen, z.B. mittels Hybridisierung nach Standardverfahren (siehe z.B. Sambrook et al., 1989, Molecular Cloning, A Laboratory Manual, 2. Aufl. Cold Spring Harbor Laboratory Press, Cold Spring Harbor, NY).
Als Hybridisierungsprobe können z.B. Nucleinsäuremoleküle verwendet werden, die exakt die oder im wesentlichen die unter Seq ID No. 1 angegebene Sequenz oder Teile dieser Sequenz aufweisen. Bei den als Hybridisierungsprobe verwendeten DNA-Fragmenten kann es sich auch um synthetische DNA-Fragmente handeln, die mit Hilfe der gängigen DNA-Synthesetechniken hergestellt wurden und deren Sequenz im wesentlichen mit der der erfindungsgemäßen Nucleinsäuremoleküle übereinstimmt. Hat man Gene identifiziert und isoliert, die mit den erfindungsgemäßen Sequenzen hybridisieren, ist eine Bestimmung der Sequenz und eine Analyse der Eigenschaften der von dieser Sequenz codierten Proteine erforderlich.

Weiterhin betrifft die vorliegende Erfindung Nucleinsäuremoleküle, deren Sequenzen aufgrund des genetischen Codes degeneriert sind im Vergleich zu den Sequenzen der obengenannten Moleküle, und die ein Protein codieren, das in den Plastiden pflanzlicher Zellen teilweise an Stärkekörner gebunden vorliegt.

Gegenstand der Erfindung sind ebenfalls Fragmente, Derivate und allelische Varianten der oben beschriebenen Nucleinsäuremoleküle, die das oben beschriebene Protein codieren. Unter Fragmenten werden dabei Teile der Nucleinsäuremoleküle verstanden, die lang genug sind, um das beschriebene Protein zu codieren. Der Ausdruck Derivat bedeutet in diesem Zusammenhang, daß die Sequenzen dieser Moleküle sich von den Sequenzen der oben beschriebenen Nucleinsäuremoleküle an einer oder mehreren Positionen unterscheiden und einen hohen Grad an Homologie zu den Sequen+zen dieser Moleküle aufweisen. Homologie bedeutet dabei eine Sequenzidentität von mindestens 40%, insbesondere eine Identität von mindestens 60%, vorzugsweise über 80% und besonders bevorzugt über 90%. Die Abweichungen zu den oben beschriebenen Nucleinsäuremolekülen können dabei durch Deletion, Substitution, Insertion oder Rekombination entstanden sein.
Homologie bedeutet ferner, daß funktionelle und/oder strukturelle Äquivalenz zwischen den betreffenden Nucleinsäuremolekülen oder den durch sie codierten Proteinen, besteht. Bei den Nucleinsäuremolekülen, die homolog zu den oben beschriebenen Nucleinsäuremolekülen sind und Derivate dieser Moleküle darstellen, handelt es sich in der Regel um Variationen dieser Nucleinsäuremoleküle, die Modifikationen darstellen, die dieselbe biologische Funktion ausüben. Es kann sich dabei sowohl um natürlicherweise auftretende Variationen handeln, beispielsweise um Sequenzen aus anderen Organismen, oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln.
Bei den allelischen Varianten kann es sich sowohl um natürlich auftretende Varianten handeln, als auch um synthetisch hergestellte oder durch rekombinante DNA-Techniken erzeugte Varianten.
Die von den verschiedenen Varianten der erfindungsgemäßen Nucleinsäuremoleküle codierten Proteine weisen bestimmte gemeinsame Charakteristika auf. Dazu können z.B. Enzymaktivität, Molekulargewicht, immunologische Reaktivität, Konformation etc. gehören, sowie physikalische Eigenschaften wie z.B. das Laufverhalten in Gelelektrophoresen, chromatographisches Verhalten, Sedimentationskoeffizienten, Löslichkeit, spektroskopische Eigenschaften, Stabilität, pH-Optimum, Temperatur-Optimum etc.

Die erfindungsgemäßen Nucleinsäuremoleküle können prinzipiell aus jedem Organismus stammen, der die beschriebenen Proteine exprimiert, vorzugsweise aus Pflanzen, insbesondere aus stärkesynthetisierenden bzw. stärkespeichernden Pflanzen. Besonders bevorzugt sind dabei z.B. Getreidearten (wie Gerste, Roggen, Hafer, Weizen etc.), Mais, Reis, Erbse, Maniok, Kartoffel usw. Ferner können sie durch dem Fachmann geläufige Synthesetechniken hergestellt werden.

Bei den erfindungsgemäßen Nucleinsäuremolekülen kann es sich sowohl um DNA-, beispielsweise um cDNA oder genomische DNA, als auch um RNA-Moleküle handeln.

Ferner betrifft die Erfindung Vektoren, insbesondere Plasmide, Cosmide, Viren, Bacteriophagen und andere in der Gentechnik gängige Vektoren, die die oben beschriebenen erfindungsgemäßen Nucleinsäuremoleküle enthalten.

In einer bevorzugten Ausführungsform sind die in den Vektoren enthaltenen Nucleinsäuremoleküle verknüpft mit regulatorischen Elementen, die die Transkription in prokaryontischen oder eukaryontischen Zellen gewährleisten.

In einer weiteren Ausführungsform betrifft die Erfindung Wirtszellen, insbesondere prokaryontische oder eukaryontische Zellen, die mit einem oben beschriebenen erfindungsgemäßen Nucleinsäuremolekül oder Vektor transformiert und/oder genetisch manipuliert sind, sowie Zellen, die von solchen Zellen abstammen und ein erfindungsgemäßes Nucleinsäuremolekül oder einen Vektor enthalten. Dabei handelt es sich vorzugsweise um bakterielle Zellen oder um Pflanzenzellen.

Es wurde nun gefunden, daß das durch die erfindungsgemäßen Nucleinsäuremoleküle codierte Protein einen Einfluß auf die Stärkesynthese bzw. -modifikation hat, und eine Veränderung der Menge des Proteins in pflanzlichen Zellen zu Veränderungen im Stärkemetabolismus der Pflanzen führt, insbesondere zur Synthese von Stärken mit veränderten physikalischen und chemischen Eigenschaften.
Durch die Bereitstellung der erfindungsgemäßen Nucleinsäuremoleküle ist es somit möglich, mit Hilfe gentechnischer Verfahren Pflanzen herzustellen, die eine modifizierte Stärke synthetisieren, die sich in ihrer Struktur und ihren physikalischen und chemischen Eigenschaften von in Wildtyp-Pflanzen synthetisierter Stärke unterscheidet. Hierzu werden die erfindungsgemäßen Nucleinsäuremoleküle mit regulatorischen Elementen verknüpft, die die Transkription und Translation in Pflanzenzellen gewährleisten, und in pflanzliche Zellen eingebracht.
Die vorliegende Erfindung betrifft somit auch transgene Pflanzenzellen, die ein erfindungsgemäßes Nucleinsäuremolekül enthalten, wobei dieses mit regulatorischen Elementen verknüpft ist, die die Transkription in pflanzlichen Zellen gewährleisten. Die regulatorischen Elemente sind vorzugsweise heterolog in Bezug auf das Nucleinsäuremolekül.

Die transgenen Pflanzenzellen können nach dem Fachmann bekannten Techniken zu ganzen Pflanzen regeneriert werden. Die durch Regeneration der erfindungsgemäßen transgenen Pflanzenzellen erhältlichen Pflanzen sind ebenfalls Gegenstand der vorliegenden Erfindung. Ferner sind Gegenstand der Erfindung Pflanzen, die die obenbeschriebenen transgenen Pflanzenzellen enthalten. Bei den transgenen Pflanzen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen, insbesondere stärkespeichernde Nutzpflanzen, wie z.B. Getreidearten (Roggen, Gerste, Hafer, Weizen etc.), Reis, Mais, Erbse, Maniok und Kartoffel.

Die erfindungsgemäßen transgenen Pflanzenzellen und Pflanzen synthetisieren aufgrund der Expression bzw. zusätzlichen Expression eines erfindungsgemäßen Nucleinsäuremoleküls eine Stärke, die im Vergleich zu Stärke aus Wildtyp-Pflanzen, d.h. nicht-transformierten Pflanzen, modifiziert ist, insbesondere im Hinblick auf die Viskosität wäßriger Lösungen dieser Stärke und/oder den Phosphatgehalt. Dieser ist in der Regel bei der Stärke aus transgenen Pflanzenzellen bzw. Pflanzen erhöht, wodurch die physikalische Eigenschaften der Stärke verändert werden.

Gegenstand der vorliegenden Erfindung ist somit auch die aus den erfindungsgemäßen transgenen Pflanzenzellen und Pflanzen erhältliche Stärke.

Gegenstand der Erfindung ist ebenfalls ein Verfahren zur Herstellung eines Proteins, das in pflanzlichen Zellen sowohl an Stärkekörner gebunden als auch in löslicher Form vorliegt, bei dem erfindungsgemäße Wirtszellen unter Bedingungen kultiviert werden, die die Expression des Proteins erlauben, und das Protein aus den Zellen und/oder dem Kulturmedium isoliert wird.

Ferner betrifft die Erfindung die durch die erfindungsgemä-βen Nucleinsäuremoleküle codierten Proteine sowie Proteine, die durch das oben beschriebene Verfahren erhältlich sind. Es handelt sich dabei vorzugsweise um pflanzliche, kerncodierte Proteine, die in den Plastiden lokalisiert sind. In den Plastiden liegen diese Enzyme sowohl an den Stärkekörnern gebunden vor als auch frei. Die entsprechenden Proteine aus *Solanum tuberosum* weisen in einer SDS-Gelelektrophorese ein Molekulargewicht von 140-160 kd auf und führen bei Expression in E. coli zu einer erhöhten Phosphorylierung des in den Zellen synthetisierten Glycogens.

Gegenstand der Erfindung sind ebenfalls Antikörper, die spezifisch ein erfindungsgemäßes Protein erkennen. Es kann sich hierbei sowohl um monoclonale als auch um polyclonale Antikörper handeln.

Ferner betrifft die vorliegende Erfindung Nucleinsäuremoleküle, die mit einem erfindungsgmeäßen Nucleinsäuremolekül spezifisch hybridisieren und eine Länge von mindestens 15 Nucleotiden aufweisen. Spezifisch hybridisieren bedeutet dabei, daß unter konventionellen Hybridisierungsbedingungen, vorzugsweise unter stringenten Bedingungen, keine signifikanten Kreuzhybridisierungen mit Sequenzen auftreten, die für andere Proteine codieren. Vorzugsweise haben derartige Nucleinsäuremoleküle eine Länge von mindestens 20, besonders bevorzugt von mindestens 50 und insbesondere von mindestens 100 Nucleotiden. Verwendet werden können solche Moleküle beispielsweise als PCR-Primer, als Hybridisierungsproben oder als DNA-Moleküle, die antisense-RNA codieren.

Es wurde ferner gefunden, daß es möglich ist, die Eigenschaften der in Pflanzenzellen synthetisierten Stärke dadurch zu beeinflussen, daß die Menge an Proteinen, die durch die erfindungsgemäßen Nucleinsäuremoleküle codiert werden, in den Zellen verringert wird. Diese Verringerung kann beispielsweise durch antisense-Expression der erfindungsgemäßen Nucleinsäuremoleküle, durch Expression geeigneter Ribozyme oder mittels Cosuppression erfolgen.

Gegenstand der vorliegenden Erfindung sind somit auch DNA-Moleküle, die eine antisense-RNA codieren, die komplementär ist zu Transkripten eines erfindungsgemäßen DNA-Moleküls. Komplementär bedeutet dabei, daß die codierte RNA nicht 100 % komplementär sein muß, sondern auch ein geringerer Grad an Komplementarität ausreicht, solange sie genügend hoch ist, um bei Expression in pflanzlichen Zellen die Expression eines erfindungsgemäßen Proteins zu inhibieren. Die transkribierte RNA ist vorzugsweise zumindest 90 % und besonders bevorzugt mindestens 95 % komplementär zu einem Transkript eines erfindungsgemäßen Nucleinsäuremoleküls. Um bei Transkription in pflanzlichen Zellen einen antisense-Effekt zu bewirken, sind derartige DNA-Moleküle mindestens 15 bp lang, vorzugsweise länger als 100 bp und besonders bevorzugt länger als 500 bp, jedoch in der Regel kürzer als 5000 bp, vorzugsweise kürzer als 2500 bp.

Ferner betrifft die Anmeldung auch DNA-Moleküle, die bei Expresssion in pflanzlichen Zellen zur Synthese einer RNA führen, die in den Pflanzenzellen aufgrund eines Cosuppressions-Effektes eine Verringerung der Expression erfindungsgemäßer Nucleinsäuremoleküle hervorruft, die das beschriebene Protein codieren. Das Prinzip der Cosuppression sowie die Herstellung entsprechender DNA-Sequenzen ist beispielsweise ausführlich beschrieben in der WO 90/12084. Derartige DNA-Moleküle codieren vorzugsweise eine RNA, die einen hohen Grad an Homologie zu Transkripten der erfindungsgemäßen Nucleinsäuremoleküle hat. Es ist dabei allerdings nicht unbedingt erforderlich, daß die codierte RNA in ein Protein translatierbar ist.

In einer weiteren Ausführungsform betrifft die vorliegende Erfindung DNA-Moleküle, die ein RNA-Molekül mit Ribozymaktivität codieren, das spezifisch Transkripte eines erfindungsgemäßen DNA-Moleküls spaltet.
Ribozyme sind katalytisch aktive RNA-Moleküle, die in der Lage sind, RNA-Moleküle und spezifische Zielsequenzen zu spalten. Mit Hilfe gentechnologischer Methoden ist es möglich, die Spezifität von Ribozymen zu verändern. Es existieren verschiedene Klassen von Ribozymen. Für die praktische Anwendung mit dem Ziel, das Transkript eines bestimmten Gens gezielt zu spalten, werden bevorzugt Vertreter zweier verschiedener Gruppen von Ribozymen verwendet. Die eine Gruppe wird gebildet von Ribozymen, die dem Typ der GruppeI-Intron-Ribozymen zuzuordnen sind. Die zweite Gruppe wird von Ribozymen gebildet, die als charakteristisches Strukturmerkmal ein sogenanntes "hammerhead"-Motiv aufweisen. Die spezifische Erkennung des Ziel-RNA-Moleküls kann modifiziert werden durch Änderung der Sequenzen, die dieses Motiv flankieren. Diese Sequenzen bestimmen über Basenpaarung mit Sequenzen im Zielmolekül die Stelle, an der die katalytische Reaktion und somit die Spaltung des Zielmoleküls erfolgt. Da die Sequenzanforderungen für eine effiziente Spaltung äußerst gering sind, ist es im Prinzip möglich, spezifische Ribozyme für praktisch jedes beliebige RNA-Molekül zu entwickeln.
Um DNA-Moleküle herzustellen, die ein Ribozym codieren, das spezifisch Transkripte eines erfindungsgemäßen DNA-Moleküls spaltet, wird beispielsweise eine DNA-Sequenz, die eine katalytische Domäne eines Ribozyms codiert, beiderseits mit DNA-Sequenzen verknüpft, die homolog sind zu Sequenzen des Zielenzyms. Als Sequenzen, die die katalytische Domänen codieren, kommen beispielsweise in Frage die katalytische Domäne der Satelliten-DNA des SCMo-Virus (Davies et al., Virology 177 (1990), 216-224) oder die der Satelliten-DNA des TobR-Virus (Steinecke et al., EMBO J. 11 (1992), 1525-1530; Haseloff und Gerlach, Nature 334 (1988), 585-591). Die die katalytische Domäne flankierenden DNA-Sequenzen stammen vorzugsweise aus den oben beschriebenen erfindungsgemäßen DNA-Molekülen.

In einer weiteren Ausführungsform betrifft die vorliegende Erfindung Vektoren, die die oben beschriebenen DNA-Moleküle enthalten, insbesondere solche, bei denen die beschriebenen DNA-Moleküle verknüpft sind mit regulatorischen Elementen, die die Transkription in pflanzlichen Zellen gewährleisten.

Ferner betrifft die vorliegende Erfindung Wirtszellen, die die beschriebenen DNA-Moleküle oder Vektoren enthalten. Die Wirtszelle kann eine prokaryontische, beispielsweise bakterielle, oder eukaryontische Zelle sein. Bei den eukaryontischen Wirtszellen handelt es sich vorzugsweise um pflanzliche Zellen.

Ferner betrifft die Erfindung transgene Pflanzenzellen, die ein oben beschriebenes DNA-Molekül enthalten, das eine anti-sense-RNA, ein Ribozym oder eine RNA, die zu einem Cosuppressions-Effekt führt, codiert, wobei dieses DNA-Molekül verknüpft ist mit DNA-Elementen, die die Transkription in pflanzlichen Zellen gewährleisten. Diese transgenen Pflanzenzellen können nach gängigen Techniken zu ganzen Pflanzen regeneriert werden. Die Erfindung betrifft somit auch Pflanzen, die erhältlich sind durch Regeneration aus den beschriebenen transgenen Pflanzenzellen, sowie Pflanzen, die die beschriebenen transgenen Pflanzenzellen enthalten. Bei den transgenen Pflanzen kann es sich wiederum um Pflanzen jeder beliebigen Pflanzenspezies handeln, vorzugsweise um Nutzpflanzen, insbesondere stärkespeichernde, wie oben angegeben.

Durch die Expression der beschriebenen DNA-Moleküle, die antisense-RNA, ein Ribozym oder eine "Cosuppressions-RNA" codieren, in den transgenen Pflanzenzellen kommt es zu einer Verringerung der Menge an Proteinen, die durch erfindungsgemäße DNA-Moleküle codiert werden, die endogen in den Zellen vorliegen. Diese Verringerung hat überraschenderweise eine drastische Veränderung der physikalischen und chemischen Eigenschaften der in den Pflanzenzellen synthetisierten Stärke zur Folge, insbesondere der Viskositätseigenschaften wäßriger Lösungen dieser Stärke, des Phosphatgehaltes als auch der Freisetzung reduzierender Zucker bei Lagerung der Pflanzenzellen oder Pflanzenteile bei niedrigen Temperaturen. Die Eigenschaften der in den transgenen Pflanzenzellen synthetisierten Stärke wird weiter unten ausführlich beschrieben.

Gegenstand der vorliegenden Erfindung ist somit auch die aus den beschriebenen transgenen Pflanzenzellen und Pflanzen erhältliche Stärke.

Ferner betrifft die Erfindung die durch die beschriebenen DNA-Moleküle codierten antisense-RNA-Moleküle, sowie RNA-Moleküle mit Ribozymaktivität und RNA-Moleküle, die einen Cosuppressions-Effekt hervorrufen, die durch Transkription erhältlich sind.

Ein weiterer Gegenstand der vorliegenden Erfindung ist ein Verfahren zur Herstellung transgener Pflanzenzellen, die im Vergleich zu nicht-transformierten Zellen eine modifizierte Stärke synthetisieren, bei dem in den Pflanzenzellen die Menge an Proteinen verringert wird, die durch erfindungsgemäße DNA-Moleküle codiert werden, die endogen in den Zellen vorliegen.

In einer bevorzugten Ausführungsform erfolgt diese Verringerung mit Hilfe eines antisense-Effektes. Hierzu werden erfindungsgemäße DNA-Moleküle oder Teile davon in antisense-Orientierung mit einem Promotor verknüpft, der die Transkription in pflanzlichen Zellen gewährleistet, sowie gegebenenfalls mit einem Terminationssignal, das die Termination der Transkription sowie die Polyadenylierung des Transkriptes gewährleistet. Um einen effizienten antisense-Effekt in den pflanzlichen Zellen zu gewährleisten, sollte die synthetisierte antisense-RNA eine Mindestlänge von 15 Nukleotiden, vorzugsweise von mindestens 100 Nukleotiden und besonders bevorzugt von über 500 Nukleotiden aufweisen. Ferner sollte die die antisense-RNA codierende DNA-Sequenz in bezug auf die zu transformierende Pflanzenspezies homolog sein. Es können jedoch auch DNA-Sequenzen verwendet werden, die einen hohen Grad an Homologie zu endogen in den Zellen vorhandenen DNA-Sequenzen aufweisen, vorzugsweise eine Homologie von mehr als 90%, besonders bevorzugt von mehr als 95%.

In einer weiteren bevorzugten Ausführungsform erfolgt die Verringerung der Menge an Proteinen, die durch die erfindungsgemäBen DNA-Moleküle codiert werden, durch einen Ribozym-Effekt. Die prinzipielle Wirkungsweise von Ribozymen, sowie die Konstruktion von DNA-Molekülen, die derartige RNA-Moleküle codieren, wurden bereits oben beschrieben. Um in transgenen Zellen eine RNA mit Ribozymaktivität zu exprimieren, werden die oben beschriebenen DNA-Moleküle, die für ein Ribozym codieren, mit DNA-Elementen verknüpft, die die Transkription in pflanzlichen Zellen gewährleisten, insbesondere mit einem Promotor und einem Terminationssignal. Die in den Pflanzenzellen synthetisierten Ribozyme führen zur Spaltung von Transkripten von erfindungsgemäßen DNA-Molekülen, die endogen in den Zellen vorliegen.

Eine weitere Möglichkeit der Verringerung der Menge an Proteinen, die durch die erfindungsgemäßen Nucleinsäuremoleküle codiert werden, ist die Cosuppression. Gegenstand der Erfindung sind dabei auch die durch das erfindungsgemäße Verfahren erhältlichen Pflanzenzellen, die dadurch charakterisiert sind, daß bei ihnen die Menge an Proteinen verringert ist, die durch die erfindungsgemäßen DNA-Moleküle codiert werden, und die im Vergleich zu Wildtyp-Zellen eine modifizierte Stärke synthetisieren.

Ferner betrifft die Erfindung Pflanzen, die erhältlich sind durch Regeneration der beschriebenen Pflanzenzellen, sowie Pflanzen, die die beschriebenen erfindungsgemäßen Zellen enthalten.

Die aus den beschriebenen Pflanzenzellen und Pflanzen erhältliche Stärke ist ebenfalls Gegenstand der vorliegenden Erfindung. Diese weist im Vergleich zu Stärke aus Wildtyp-Pflanzen veränderte physikalische und chemische Eigenschaften auf. Beispielsweise besitzt diese Stärke im Vergleich zur Stärke aus Wildtyp-Pflanzen einen reduzierten Phosphatgehalt. Ferner zeigen wäßrige Lösungen dieser Stärke veränderte Viskositätseigenschaften.

In einer bevorzugten Ausführungsform ist der Phosphatgehalt der beschriebenen Stärke um mindestens 50%, vorzugsweise um mindestens 75% und besonders bevorzugt um mehr als 80% im Vergleich zu Stärke aus Wildtyp-Pflanzen verringert.

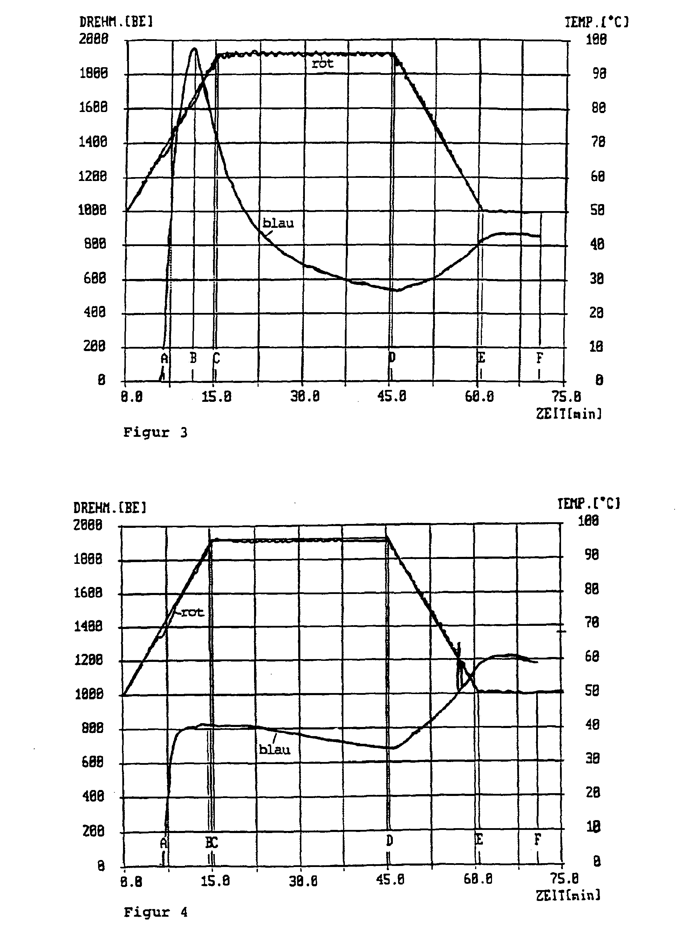

Der besondere Vorzug der beschriebenen Stärke liegt in den veränderten Viskositätseigenschaften wäßriger Lösungen dieser Stärke.
Ein gängiger Test, der verwendet wird, um die Viskositätseigenschaften zu bestimmen, ist der sogenannte Brabender-Test.
Dieser Test wird durchgeführt unter der Verwendung eines Apparates, der beispielsweise als Viskograph E bekannt ist. Hergestellt und vertrieben wird dieses Instrument unter anderem von der Firma Brabender OHG Duisburg (Deutschland).
Der Test besteht im wesentlichen darin, daß Stärke in Gegenwart von Wasser zunächst erhitzt wird, um zu bestimmen, wann die Hydratisierung und das Schwellen der Stärkekörner einsetzt. Dieser Vorgang, der auch als Gelatinisierung bzw.
Verkleisterung bezeichnet wird, beruht auf der Auflösung von Wasserstoffbrückenbindungen und geht einher mit einer meßbaren Viskositätszunahme der Stärkesuspension. Während eine weitere Erhitzung nach der Gelatinisierung zur vollständigen Auflösung der Stärkepartikel und einer Abnahme der viskosität führt, kommt es bei einer Abkühlung unmittelbar nach der Gelatinisierung typischerweise zu einer Viskositätszunahme (siehe Fig. 3). Das Resultat eines Brabendertests ist eine Kurve, die die Viskosität in Abhängigkeit von der Zeit angibt, wobei zunächst eine Temperaturzunahme bis über die Gelatinisierungstemperatur und anschließend eine Abkühlung erfolgt.
Die Analyse einer Brabender-Kurve zielt in der Regel ab auf die Bestimmung der Verkleisterungsstemperatur, der maximalen Viskosität bei Erhitzen, der Viskositätszunahme bei Abkühlung, sowie der Viskosität nach dem Erkalten. Diese Parameter sind wichtige Charakteristika, die die Qualität einer Stärke sowie ihre Verwendbarkeit für verschiedene Anwendungen bestimmen.

Die Stärke, die sich beispielsweise aus Kartoffelpflanzen isolieren läßt, bei denen durch einen *antisense*-Effekt die Menge an erfindungsgemäßen Proteinen in den Zellen reduziert wurde, zeigt Charakteristika, die stark von denen abweichen, die Stärke zeigt, die aus Wildtyppflanzen isolierbar ist. Im Vergleich zu diesen zeigt sie nur eine geringe Viskositätszunahme beim Erhitzen, eine geringere maximale Viskosität, sowie eine stärkere Viskositätszunahme beim Erkalten (siehe Fig. 3, 4 und 5).

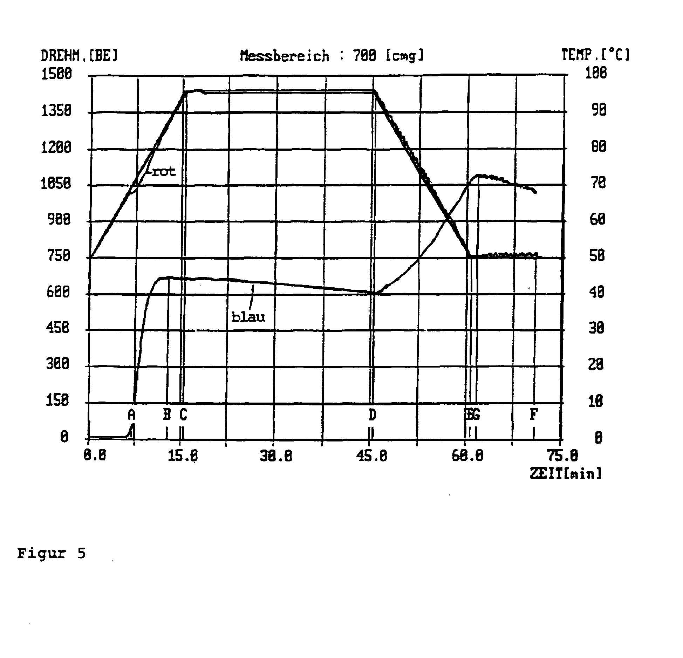

In einer bevorzugten Ausführungsform betrifft die Erfindung somit eine Stärke, deren wäßrige Lösungen die in Fig. 4 oder 5 dargestellten charakteristischen Viskositätseigenschaften besitzen. Die modifizierte Stärke weist, insbesondere unter den in Beispiel 8 a genannten Bedingungen zur Bestimmung der Viskosität mit Hilfe eines Brabender-Viskosimeters, das Charakteristikum auf, daß während des Aufkochens im Vergleich zu Wildtypstärke nur eine geringe Viskositätszunahme erfolgt. Dies bietet die Möglichkeit, die erfindungsgemäße Stärke zur Herstellung höher konzentrierter Kleister zu verwenden.
Ferner weist die modifizierte Stärke die Eigenschaft auf, daß es nach Erreichen der maximalen Vikosität nur zu einer geringen Viskositätsabnahme kommt. Dagegen kommt es bei einem Erkalten zu einer starken Viskositätszunahme, so daß die Viskosität höher ist als die einer Stärke aus Wildtyp-Pflanzen.
Weiterhin ist es möglich, durch Verringerung der Menge an erfindungsgemäßen Proteinen in transgenen Pflanzenzellen eine Stärke herzustellen, die bei Lagerung von Pflanzenteilen, die diese Stärke enthalten, bei niedrigen Temperaturen, insbesondere bei 4-8 °C, zu einer veringerten Freisetzung reduzierender Zucker führt im Vergleich zu Stärke aus nicht-transformierten Zellen. Diese Eigenschaft ist beispielsweise besonders vorteilhaft für die Bereitstellung von Kartoffeln, die bei Lagerung bei niedrigen Temperaturen eine veringerte Freisetzung von reduzierenden Zuckern aufweisen, d.h. ein verringertes "cold sweetening". Derartige Kartoffeln sind besonders gut geeignet zur Herstellung von Pommes frites, Chips oder ähnlichem, da bei ihrer Verwendung unerwünschte Bräunungsreaktionen (Maillard-Reaktionen) ausbleiben oder zumindestens stark verringert sind.

In einer besonders bevorzugten Ausführungsform der vorliegenden Erfindung wird in den transformierten Pflanzenzellen nicht nur die Synthese eines erfindungsgemäßen Proteins reduziert, sondern darüber hinaus auch die Synthese mindestens eines weiteren, an der Särkesynthese und/oder Modifikation beteitligten Enzyms. Bevorzugt sind dabei beispielsweise stärkekorngebundene Stärkesynthasen oder verzweigungsenzyme. Es wurde überraschend gefunden, daß Kartoffelpflanzen, bei denen die Synthese des erfindungsgemäßen Proteins sowie auch des Verzweigungsenzyms aufgrund eines *antisense*-Effekts reduziert ist, eine Stärke synthetisieren, die in ihren Eigenschaften stark abweicht von Stärke aus Wildtyppflanzen.

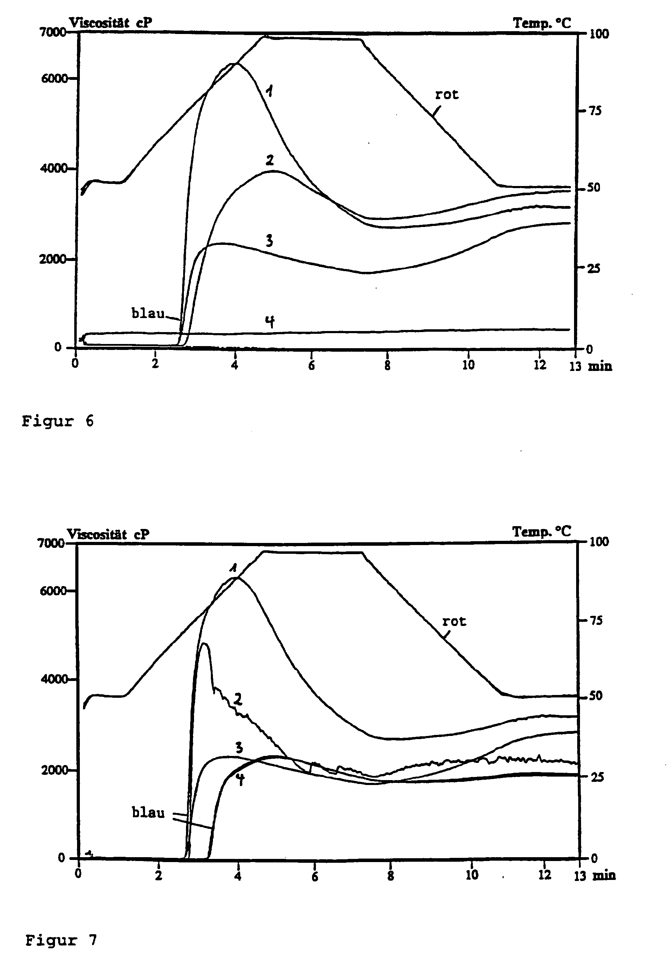

Im Vergleich zu Wildtypstärke zeigen wäßrige Lösungen dieser modifizierten Stärke so gut wie keine Viskositätszunahme beim Erhitzen oder beim Abkühlen (vgl. Fig. 6).
Des weiteren zeigt eine mikroskopische Analyse der Stärkekörner vor und nach dem Erhitzen deutlich, daß im Gegensatz zur Wildtypstärke die Stärkekörner aus derart veränderten Pflanzen nicht aufgeschlossen sind, sondern ihre ursprüngliche Struktur annähernd beibehalten. Es handelt sich somit um eine gegenüber dem Kochprozeß resistente Stärke. Wird von dieser Stärke der Amylosegehalt bestimmt mittels der in dem Beispielsteil beschriebenen Methodik, so ergeben sich Amylosegehalte von über 50 %, vorzugsweise über 60 % und besonders bevorzugt über 70 % für diese Stärke. Die wäßrigen Lösungen der aus diesen Pflanzen isolierbaren Stärke zeigen vorzugsweise die in Fig. 6 dargestellten charakteristischen Viskositätseigenschaften.

Eine derartige erfindungsgemäße hochamylosehaltige Stärke weist gegenüber Wildtypstärke eine Reihe von Vorteilen für verschiedene Verwendungen auf. So besitzen hochamylosehaltige Stärken ein hohes Potential zur Nutzung in Folien und Filmen. Die auf der Grundlage von hochamylosehaltigen Stärken erzeugten Folien und Filme, die in weitesten Bereichen der Verpackungsindustrie eingesetzt werden können, besitzen den deutlichen Vorteil, daß sie biodegradierbar sind. Neben dieser Anwendung, die im wesentlichen von in klassischer Weise auf der Erdölchemie basierenden Polymeren abgedeckt wird, besitzt die Amylose noch weitere unikate Anwendungsfelder, die durch die Eigenschaft der Amylose bedingt sind, eine Helix zu bilden. Die von der Amylose gebildete Helix ist im Inneren hydrophob und außen hydrophil. Aufgrund dessen kann Amylose zur Komplexierung und molekularen Verkapselung niedermolekularer oder auch höher molekularer Substanzen eingesetzt werden. Beispiele dafür sind:
- die molekulare Verkapselung von Vitaminen und Wirkstoffen mit dem Ziel des Schutzes vor Oxydation, Verflüchtigung, thermischem Abbau oder aber der Überführung in ein wäßriges Milieu;
- die molekulare Verkapselung von Aromastoffen zur Erhöhung der Löslichkeit;
- die molekulare Verkapselung von Düngemitteln/Pestiziden zur Stabilisierung und kontrollierten Freisetzung;
- die molekulare Verkapselung von Arzneistoffen zur Stabilisierung der Dosierbarkeit und kontrollierten Freisetzung von Retardpräparaten.

Eine weitere wichtige Eigenschaft von Amylose ist die Tatsache, daß es sich um ein chirales Molekül handelt. Aufgrund der Chiralität kann es präferentiell nach Immobilisierung beispielsweise an einer Säule zur Trennung von Enantiomeren eingesetzt werden.

Weiterhin wurde überraschend gefunden, daß Stärke, die sich aus Kartoffelpflanzen isolieren läßt, bei denen durch einen antisense-Effekt die Menge an erfindungsgemäßen Proteinen in den Zellen reduziert wird, in Kombination mit einer Reduktion der Proteine, die die enzymatische Aktivität einer stärkekorngebundenen Stärkesynthase der Isoform I (GBSSI) aufweisen, Charakteristika zeigt, die stark von denen abweichen, die Stärke zeigt, die aus Wildtyppflanzen isolierbar ist. Im Vergleich zu Stärke aus Wildtyppflanzen zeigen wäßrige Lösungen dieser Stärke nur eine geringe Viskositätszunahme beim Erhitzen, eine geringere maximale Viskosität sowie so gut wie keine Viskositätszunahme beim Erkalten (vgl.
Fig. 7). Wenn von dieser Stärke das Verhältnis Amylose zu Amylopektin bestimmt wird, zeichnet sich diese Stärke dadurch aus, daß fast keine Amylose mehr nachweisbar ist. Der Amylosegehalt dieser Stärke liegt vorzugsweise unter 5%, besonders bevorzugt unter 2 %. Die erfindungsgemäße Stärke unterscheidet sich ferner von der bekannten Stärke, die durch Inhibierung des GBSSI-Gens alleine mittels gentechnischer Verfahren in transgenen Kartoffelpflanzen erzeugt werden kann. So weist diese Stärke eine starke Viskositätszunahme beim Erhitzen auf. Die wäßrigen Lösungen der erfindungsgemä-βen Stärke zeigen vorzugsweise die in Fig. 7 dargestellten charakteristischen Viskositätseigenschaften. Insbesondere unter den im Beispiel 13 genannten Bedingungen zur Bestimmung der Viskosität mit Hilfe eines Rapid Visco Analysers weist die modifizierte Stärke das Charakteristikum auf, daß während des Aufkochens im Vergleich zur Wildtypstärke, aber auch im Vergleich zur waxy- Stärke nur eine geringe Viskositätszunahme erfolgt. Dies bietet die Möglichkeit, die erfindungsgemäße Stärke zur Herstellung höher konzentrierter Kleister zu verwenden. Ferner weist die modifizierte Stärke die Eigenschaft auf, daß es nach Erreichen der maximalen Viskosität nur zu einer geringeren Viskositätsabnahme kommt sowie zu so gut wie keiner Viskositätszunahme beim Erkalten.

Möglichkeiten zur Verringerung der Aktivität eines Verzweigungsenzyms in pflanzlichen Zellen wurden bereits beschrieben, beispielsweise in der WO 92/14827 und der WO 95/26407. Die Verringerung der Aktivität einer stärkekorngebundenen Stärkesynthase der Isoform I (GBSSI) kann unter der Verwendung von dem Fachmann bekannten Methoden erfolgen, beispielsweise mittels eines *antisense*-Effekts. DNA-Sequenzen, die eine GBSSI aus Kartoffel codieren, sind beispielsweise bekannt aus Hergersberg (Dissertation (1988) Universität Köln, Visser et al. (Plant Sci. 64 (1989), 185-192) oder van der Leiy et al. (Mol. Gen. Genet. 228 (1991), 240-248).

Das erfindungsgemäße Verfahren kann prinzipiell auf alle Pflanzenspezies angewendet werden. Von Interesse sind sowohl monokotyle als auch dikotyle Pflanzen, insbesondere Nutzpflanzen und hierbei bevorzugt stärkespeichernde Pflanzen, wie z.B. Getreidepflanzen (Roggen, Gerste, Hafer, Weizen, etc.), Reis, Mais, Erbse, Maniok und Kartoffel.

Unter dem Begriff "regulatorische DNA-Elemente, die die Transkription in pflanzlichen Zellen gewährleisten" werden im Rahmen der vorliegenden Erfindung DNA-Abschnitte verstanden, die die Initiation bzw. die Termination der Transkription in pflanzlichen Zellen ermöglichen. Zu den DNA-Abschnitten, die die Initiation der Transkription gewährleisten zählen insbesondere Promotoren.
Für die Expression der verschiedenen oben beschriebenen erfindungsgemäßen DNA-Moleküle in Pflanzen kommt im Prinzip jeder in pflanzlichen Zellen funktionale Promotor in Betracht. Der Promotor kann homolog oder heterolog in bezug auf die verwendete Pflanzenspezies sein. Geeignet ist beispielsweise der 35S-Promotor des Cauliflower-Mosaik-Virus (Odell et al., Nature 313 (1985), 810-812), der eine konstitutive Expression in allen Geweben einer Pflanze gewährleistet und das in der WO/9401571 beschriebene Promotorkonstrukt. Es können jedoch auch Promotoren verwendet werden, die nur zu einem durch äußere Einflüsse determinierten Zeitpunkt (siehe beispielsweise WO/9307279) oder in einem bestimmten Gewebe der Pflanze zu einer Expression nachfolgender Sequenzen führen (siehe z. B. Stockhaus et al., EMBO J. 8 (1989), 2245-2251). Präferentiell werden Promotoren eingesetzt, die in den stärkespeichernden Organen der zu transformierenden Pflanzen aktiv sind. Dies sind beim Mais die Maiskörner, während es bei der Kartoffel die Knollen sind. Zur Transformation der Kartoffel kann insbesondere, aber nicht ausschließlich, der knollenspezifische B33-Promotor (Rocha-Sosa et al., EMBO J. 8 (1989), 23-29) verwendet werden.
Neben Promotoren können DNA-Abschnitte zur Initiation der Transkription auch DNA-Sequenzen enthalten, die eine weitere Steigerung der Transkription gewährleisten, beispielsweise sogenannte Enhancer-Elemente.
Ferner kann der Begriff "regulatorische DNA-Elemente" auch Terminationssignale umfassen, die der korrekten Beendigung der Transkription sowie der Addition eines Poly-A-Schwanzes an das Transkript dienen, dem eine Funktion bei der Stabilisierung der Transkripte beigemessen wird. Derartige Elemente sind in der Literatur beschrieben und sind beliebig austauschbar. Beispiele für derartige Terminationssequenzen sind die 3'-nichttranslatierten Regionen, die das Polyadenylierungssignal des Nopalin-Synthase-Gens (NOS-Gen) oder des Octopinsynthase-Gens (Gielen et al., EMBO J. 8 (1989), 23-29) aus Agrobakterien umfassen, oder die 3'-nichttranslatierten Regionen der Gene der Speicherproteine aus Sojabohne, sowie die der Gene der kleinen Untereinheit der Ribulose-1,5-Bisphosphat-Carboxylase (ssRUBISCO).

Die Einführung erfindungsgemäßer DNA-Moleküle in pflanzliche Zellen erfolgt vorzugsweise unter Verwendung von Plasmiden. Vorzugsweise werden dafür Plasmide verwendet, die eine stabile Integration der eingeführten DNA in das pflanzliche Genom gewährleisten.
In den Beispielen der vorliegenden Erfindung wird der binäre Vektor pBinAR (Höfgen und Willmitzer, Plant Sci. 66 (1990), 221-230) verwendet. Bei diesem Vektor handelt es sich um ein Derivat des binären Vektors pBin19 (Bevan, Nucl. Acids Res. 12 (1984), 8711-8721), der kommerziell erhältlich ist (Clontech Laboratories, Inc., USA).
Es ist jedoch auch jeder andere Pflanzentransformationsvektor geeignet, in den eine Expressionskassette inseriert werden kann, und der die Integration der Expressionskassette in das pflanzliche Genom gewährleistet.
Zur Vorbereitung der Einführung fremder Gene in höhere Pflanzen stehen eine große Anzahl von Klonierungsvektoren zur Verfügung, die ein Replikationssignal für *E.coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltene Plasmid wird für die Transformation von *E.coli*-Zellen verwendet. Transformierte *E.coli*-Zellen werden in einem geeigneten Medium kultiviert, anschließend geerntet und lysiert. Das Plasmid wird nach Standardmethoden wiedergewonnen. Als Analysemethode zur Charakterisierung der gewonnenen Plasmid-DNA werden im allgemeinen Restriktionsanalysen und Sequenzanalysen eingesetzt. Nach jeder Manipulation kann die Plasmid DNA gespalten werden und resultierende DNA-Fragmente mit anderen DNA-Sequenzen verknüpft werden.
Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von Agrobacterium *tumefaciens* oder *Agrobacterium rhizogenes* als Transformationsmittel, die Fusion von Protoplasten, die Injektion, die Elektroporation von DNA, die Einbringung von DNA mittels der biolistischen Methode sowie weitere Möglichkeiten.
Bei der Injektion und Elektroporation von DNA in Pflanzenzellen werden an sich keine speziellen Anforderungen an die verwendeten Plasmide gestellt. Es können einfache Plasmide wie z.B. pUC-Derivate verwendet werden. Sollen aber aus derartig transformierten Zellen ganze Pflanzen regeneriert werden, ist die Anwesenheit eines selektierbaren Markergens notwendig.
Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der Ti- und Ri-Plasmid T-DNA als Flankenbereich mit den einzuführenden Genen verbunden werden.
Werden für die Transformation Agrobakterien verwendet, muß die einzuführende DNA in spezielle Plasmide kloniert werden, und zwar entweder in einen intermediären Vektor oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in *E.coli* als auch in Agrobakterien replizieren. Sie enthalten ein Selektionsmarker-Gen und einen Linker oder Polylinker, welche von der rechten und linken T-DNA Grenzregion eingerahmt werden. Sie können direkt in die Agrobakterien transformiert werden (Holsters et al., Mol. Gen. Genet. 163 (1978), 181-187). Die zur Transformation der Agrobakterien verwendeten Plasmide enthalten weiterhin ein Selektionsmarkergen, beispielsweise das NPT II-Gen, das die Selektion transformierter Bakterein erlaubt. Das als Wirtszelle dienende Agrobakterium soll ein Plasmid, das eine vir-Region trägt, enthalten. Die *vir-*Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet.
Die Verwendung von T-DNA für die Transformation von Pflanzenzellen ist intensiv untersucht und ausreichend in EP 120516; Hoekema, In: The Binary Plant Vector System Offsetdrukkerij Kanters B.V., Alblasserdam (1985), Chapter V; Fraley et al., Crit. Rev. Plant. Sci., 4: 1-46 und An et al., EMBO J. 4 (1985), 277-287 beschrieben worden. Binäre Vektoren sind bereits z.T. kommerziell erhältlich, z.B. pBIN19 (Clontech Laboratories, Inc., USA).
Für den Transfer der DNA in die Pflanzenzelle können Pflanzen-Explantate zweckmäßigerweise mit *Agrobacterium* tumefaciens oder Agrobacterium rhizogenes kokultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden. Die so erhaltenen Pflanzen können dann auf Anwesenheit der eingeführten DNA untersucht werden.
Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin oder Phosphinotricin u.a. vermittelt. Der individuelle gewählte Marker sollte daher die Selektion transformierter Zellen gegenüber Zellen, denen die eingeführte DNA fehlt, gestatten.
Die transformierten Zellen wachsen innerhalb der Pflanze in der üblichen Weise (siehe auch McCormick et al., Plant Cell Reports 5 (1986), 81-84). Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotypischen Eigenschaften.
Es sollten zwei oder mehrere Generationen angezogen werden, um sicherzustellen, daß das phänotypische Merkmal stabil beibehalten und vererbt wird. Auch sollten Samen geerntet werden, um sicherzustellen, daß der entsprechende Phänotyp oder andere Eigenarten erhalten geblieben sind.

Die aus den erfindungsgemäßen Pflanzenzellen bzw. Pflanzen erhältliche Stärke eignet sich aufgrund ihrer Eigenschaften neben den bereits oben angesprochenen speziellen Verwendungszwecken für verschiedene industrielle Verwendungen.
Grundsätzlich läßt sich Stärke in zwei große Kategorien unterteilen, die Hydrolyseprodukte der Stärke und die sogenannten nativen Stärken. Zu den Hydrolyseprodukten zählen im wesentliche die über enzymatische oder chemische Verfahren erhaltene Glucose sowie Glucosebausteine, die für weitere Prozesse, wie Fermentation, oder auch weitere chemische Modifikationen eingesetzt werden können. In diesem Bereich kann die Einfachheit und kostengünstige Ausführung eines Hydrolyseverfahrens, wie es gegenwärtig im wesentlichen enzymatisch unter Verwendung von Amyloglucosidase verläuft, von Bedeutung sein. Darunter läßt sich vorstellen, daß ein geringerer Einsatz von für die Hydrolyse eingesetzten Enzymen durch Veränderung der Struktur der Stärke, z.B. größere Oberfläche des Korns, leichtere Verdaulichkeit durch geringeren Verzweigungsgrad oder sterische, die Zugänglichkeit für die eingesetzten Enzyme limitierende Struktur, zu einer Kosteneinsparung führen kann. Die Verwendungen der sogenannten nativen Stärken, die wegen ihrer polymeren Struktur eingesetzt werden, lassen sich in zwei große Bereiche unterteilen:
(a) Verwendung im Nahrungsmittelbereich
   Stärke ist ein klassischer Zusatzstoff für viele Nahrungsmittel, bei denen sie im wesentlichen die Funktion des Bindens von wäßrigen Zusatzstoffen übernimmt bzw. eine Erhöhung der Viskosität oder aber eine erhöhte Gelbildung hervorruft. Wichtige Eigenschaftsmerkmale sind das Fließ- und Sorptionsverhalten, die Quell- und Verkleisterungs-temperatur, die Viskosität und Dickungsleistung, die Löslichkeit der Stärke, die Transparenz und Kleisterstruktur, die Hitze-, Scher- und Säurestabilität, die Neigung zur Retrogradation, die Fähigkeit zur Filmbildung, die Gefrier/Taustabilität, die Verdaulichkeit sowie die Fähigkeit zur Komplexbildung mit z.B. anorganischen oder organischen Ionen.
(b) Einsatz im Nicht-Nahrungsmittelbereich
   Der andere große Einsatzbereich liegt bei der Verwendung der Stärke als Hilfsstoff bei unterschiedlichen Herstellungsprozessen bzw. als Zusatzstoff in technischen Produkten. Der wesentliche Einsatzbereich für die Verwendung von Stärke als Hilfsstoff ist zum einen die Papier- und Pappeindustrie. Stärke wird dabei in erster Linie zur Retardation (Zurückhaltung von Feststoffen), der Abbindung von Füllstoff- und Feinstoffteilchen, als Festigungsstoff und zur Entwässerung eingesetzt. Darüberhinaus werden die günstigen Eigenschaften der Stärke in bezug auf die Steifigkeit, die Härte, den Klang, den
   Griff, den Glanz, die Glätte, die Spaltfestigkeit sowie die Oberflächen ausgenutzt.
   Innerhalb des Papierherstellungsprozesses sind vier Anwendungsbereiche, nämlich Oberfläche, Strich, Masse und Sprühen, zu unterscheiden.
   Die Anforderungen an die Stärke in bezug auf die Oberflächenbehandlung sind im wesentlichen ein hoher Weißegrad, eine angepaßte Viskosität, eine hohe Viskositätsstabilität, eine gute Filmbildung sowie eine geringe Staubbildung. Bei der Verwendung im Strich ist der Feststoffgehalt, eine angepaßte Viskosität, ein hohes Bindevermögen sowie eine hohe Pigmentaffinität wichtig. Als Zusatz zur Masse ist eine rasche, gleichmäßige, verlustfreie Verteilung, eine hohe mechanische Stabilität und eine vollständige Zurückhaltung im Papierfließ von Bedeutung. Beim Einsatz der Stärke im Sprühbereich sind ebenfalls ein angepaßter Feststoffgehalt, hohe Viskosität sowie ein hohes Bindevermögen von Bedeutung.
   Ein großer Einsatzbereich besteht beispielsweise in der Klebstoffindustrie, wo man die Einsatzmöglichkeiten in vier Teilbereiche gliedert: die Verwendung als reinem Stärkeleim, die Verwendung bei mit speziellen Chemikalien aufbereiteten Stärkeleimen, die Verwendung von Stärke als Zusatz zu synthetischen Harzen und Polymerdispersionen sowie die Verwendung von Stärken als Streckmittel für synthetische Klebstoffe. 90% der Klebstoffe auf Stärkebasis werden in den Bereichen Wellpappenherstellung, Herstellung von Papiersäcken, Beuteln oder Tüten, Herstellung von Verbundmaterialien für Papier und Aluminium, Herstellung von Kartonagen und Wiederbefeuchtungsleim für Briefumschläge, Briefmarken usw. eingesetzt.
   Eine weitere mögliche Verwendung als Hilfsmittel und Zusatzstoff besteht bei der Herstellung von Textilien und Textilpflegemitteln. Innerhalb der Textilindustrie sind die folgenden vier Einsatzbereiche zu unterscheiden: Der Einsatz der Stärke als Schlichtmittel, d.h. als Hilfsstoff zur Glättung und Stärkung des Klettverhaltens zum Schutz gegen die beim Weben angreifenden Zugkräfte sowie zur Erhöhung der Abriebfestigkeit beim Weben, als Mittel zur Textilaufrüstung vor allem nach qualitätsverschlechternden Vorbehandlungen, wie Bleichen, Färben usw., als Verdickungsmittel bei der Herstellung von Farbpasten zur Verhinderung von Farbstoffdiffusionen sowie als Zusatz zu Kettungsmitteln für Nähgarne.
   Ferner kann die Stärke als Zusatz bei Baustoffen verwendet werden. Ein Beispiel ist die Herstellung von Gipskartonplatten, bei der die im Gipsbrei vermischte Stärke mit dem Wasser verkleistert, an die Oberfläche der Gipsplatte diffundiert und dort den Karton an die Platte bindet. Weitere Einsatzbereiche sind die Beimischung zu Putz- und Mineralfasern. Bei Transportbeton kann die Stärke zur Verzögerung der Abbindung eingesetzt werden.
   Ferner bietet sich die Stärke zur Herstellung von Mitteln zur Bodenstabilisation an, die bei künstlichen Erdbewegungen zum temporären Schutz der Bodenpartikel gegenüber Wasser eingesetzt werden. Kombinationsprodukte aus Stärke und Polymeremulsionen sind nach heutiger Kenntnis in ihrer Erosions- und verkrustungsmindernden Wirkung den bisher eingesetzten Produkten gleichzusetzen, liegen preislich aber deutlich unter diesen.
   Ferner kann die Stärke in Pflanzenschutzmitteln zur Veränderung der spezifischen Eigenschaften der Präparate verwendet werden. So werden Stärken beispielsweise zur Verbesserung der Benetzung von Pflanzenschutz- und Düngemitteln, zur dosierten Freigabe der Wirkstoffe, zur Umwandlung flüssiger, flüchtiger und/oder übelriechender Wirkstoffe in mikrokristalline, stabile, formbare Substanzen, zur Mischung inkompatibler Verbindungen und zur Verlängerung der Wirkdauer durch Verminderung der Zersetzung eingesetzt.
   Ein wichtiges Einsatzgebiet besteht ferner im Bereich der Pharmaka, Medizin und Kosmetikindustrie. In der pharmazeutischen Industrie kann die Stärke als Bindemittel für Tabletten oder zur Bindemittelverdünnung in Kapseln eingesetzt werden. Weiterhin eignet sich die Stärke als Tablettensprengmittel, da sie nach dem Schlucken Flüssigkeit absorbiert und nach kurzer Zeit so weit quillt, daß der Wirkstoff freigesetzt wird. Medizinische Gleit- und Wundpuder sind weitere Anwendungsmöglichkeiten. Im Bereich der Kosmetik kann die Stärke beispielsweise als Träger von Puderzusatzsoffen, wie Düften und Salicylsäure eingesetzt werden. Ein relativ großer Anwendungsbereich für die Stärke liegt bei Zahnpasta.
   Auch die Verwendung der Stärke als Zusatzstoff zu Kohle und Briketts ist denkbar. Kohle kann mit einem Stärkezusatz quantitativ hochwertig agglomeriert bzw. brikettiert werden, wodurch ein frühzeitiges Zerfallen der Briketts verhindert wird. Der Stärkezusatz liegt bei Grillkohle zwischen 4 und 6%, bei kalorierter Kohle zwischen 0,1 und 0,5%. Desweiteren eignet sich die Stärke als Bindemittel, da durch ihren Zusatz zu Kohle und Brikett der Ausstoß schädlicher Stoffe deutlich vermindert werden kann.
   Die Stärke kann ferner bei der Erz- und Kohleschlammaufbereitung als Flockungsmittel eingesetzt werden.
   Ein weiterer Einsatzbereich besteht als Zusatz zu Gießereihilfsstoffen. Bei verschiedenen Gußverfahren werden Kerne benötigt, die aus Bindemittel-versetzten Sänden hergestellt werden. Als Bindemittel wird heute überwiegend Bentonit eingesetzt, das mit modifizierten Stärken, meist Quellstärken, versetzt ist.
   Zweck des Stärkezusatzes ist die Erhöhung der FlieBfestigkeit sowie die Verbesserung der Bindefestigkeit. Darüberhinaus können die Quellstärken weitere produktionstechnische Anforderungen, wie im kalten Wasser dispergierbar, rehydratisierbar, gut in Sand mischbar und hohes Wasserbindungsvermögen, aufweisen.
   In der Kautschukindustrie kann die Stärke zur Verbesserung der technischen und optischen Qualität eingesetzt werden. Gründe sind dabei die Verbesserung des Oberflächenglanzes, die Verbesserung des Griffs und des Aussehens. Dafür wird Stärke vor der Kaltvulkanisation auf die klebrigen gummierten Flächen von Kautschukstoffen gestreut. Ebenso kann sie für die Verbesserung der Bedruckbarkeit des Kautschuks eingesetzt werden.
   Eine weitere Einsatzmöglichkeit der modifizierten Stärke besteht bei der Herstellung von Lederersatzstoffen.
   Auf dem Kunststoffsektor zeichnen sich folgende Einsatzgebiete ab: die Einbindung von Stärkefolgeprodukten in den Verarbeitungsprozess (Stärke ist nur Füllstoff, es besteht keine direkte Bindung zwischen synthetischem Polymer und Stärke) oder alternativ die Einbindung von Stärkefolgeprodukten in die Herstellung von Polymeren (Stärke und Polymer gehen eine feste Bindung ein).
   Die Verwendung der Stärke als reinem Füllstoff ist verglichen mit den andere Stoffen wie Talkum nicht wettbewerbsfähig. Anders sieht es aus, wenn die spezifischen Stärkeeigenschaften zum Tragen kommen und hierdurch das Eigenschaftsprofil der Endprodukte deutlich verändert wird. Ein Beispiel hierfür ist die Anwendung von Stärkeprodukten bei der Verarbeitung von Thermoplasten, wie Polyethylen. Hierbei werden die Stärke und das synthestische Polymer durch Koexpression im Verhältnis von 1 : 1 zu einem 'master batch' kombiniert, aus dem mit granuliertem Polyethylen unter Anwendung herkömmlicher Verfahrenstechniken diverse Produkte hergestellt werden. Durch die Einbindung der Stärke in Polyethylenfolien kann eine erhöhte Stoffdurchlässigkeit bei Hohlkörpern, eine verbesserte Wasserdampfdurchlässigkeit, ein verbessertes Antistatik-verhalten, ein verbessertes Antiblockverhalten sowie eine verbesserte Bedruckbarkeit mit wäßrigen Farben erreicht werden.
   Eine andere Möglichkeit ist die Anwendung der Stärke in Polyurethanschäumen. Mit der Adaption der Stärkederivate sowie durch die verfahrenstechnische Optimierung ist es möglich, die Reaktion zwischen synthetischen Polymeren und den Hydroxygruppen der Stärke gezielt zu steuern.
   Das Ergebnis sind Polyurethanfolien, die durch die Anwendung von Stärke folgende Eigenschaftsprofile erhalten: eine Verringerung des Wärmeausdehnungskoeffizienten, Verringerung des Schrumpfverhaltens, Verbesserung des Druck/Spannungsverhaltens, Zunahme der Wasserdampfdurchlässigkeit ohne Veränderung der Wasseraufnahme, Verringerung der Entflammbarkeit und der Aufrißdichte, kein Abtropfen brennbarer Teile, Halogenfreiheit und verminderte Alterung. Nachteile, die gegenwärtig noch vorhanden sind, sind verringerte Druckfestigkeit sowie eine verringerte Schlagfestigkeit.
   Möglich ist nicht nur die Produktentwicklung von Folien. Auch feste Kunststoffprodukte, wie Töpfe, Platten und Schalen sind mit einem Stärkegehalt von über 50% herzustellen. Ferner weisen die Stärke/Polymermischungen den Vorteil auf, daß sie eine sehr viel höhere biologische Abbaubarkeit aufweisen.
   Außerordentliche Bedeutung haben weiterhin aufgrund ihres extremen Wasserbindungsvermögens Stärkepfropfpolymerisate gewonnen. Dies sind Produkte mit einem Rückgrat aus Stärke und einer nach dem Prinzip des Radikalkettenmechanismus aufgepfropften Seitengitters eines synthetischen Monomers. Die heute verfügbaren Stärkepfropfenpolymerisate zeichnen sich durch ein besseres Binde- und Rückhaltevermögen von bis zu 1000 g Wasser pro g Stärke bei hoher Viskosität aus. Diese Superabsorber werden hauptsächlich im Hygienebereich verwendet, z.B. bei Produkten wie Windeln und Unterlagen sowie im landwirtschaftlichen Sektor, z.B. bei Saatgutpillierungen.

Entscheidend für den Einsatz der neuen gentechnischen veränderten Stärke sind zum einen die Struktur, Wassergehalt, Proteingehalt, Lipidgehalt, Fasergehalt, Asche/Phosphatgehalt, Amylose/Amylopektinverhältnis, Molmassenverteilung, Verzweigungsgrad, Korngröße und -form sowie Kristallisation, zum anderen auch die Eigenschaften, die in folgenden Merkmalen münden: Fließ- und Sorptionsverhalten, Verkleisterungstemperatur, Dickungsleistung, Löslichkeit, Kleisterstruktur, Transparenz, Hitze-, Scher- und Säurestabilität, Retrogradationsneigung, Gelbildung, Gefrier/Taustabilität, Komplexbildung, Jodbindung, Filmbildung, Klebekraft, Enzymstabilität, Verdaulichkeit und Reaktivität. Besonders hervorzuheben ist ferner die viskosität.

Ferner kann die aus den erfindungsgemäßen Pflanzenzellen bzw. Pflanzen erhältliche modifizierte Stärke weiteren chemischen Modifikationen unterworfen werden, was zu weiteren Verbesserungen der Qualität für bestimmte der oben beschriebenen Einsatzgebiete führt oder zu neuen Einsatzgebieten. Diese chemischen Modifikationen sind dem Fachmann grundsätzlich bekannt. Insbesondere handelt es sich dabei um Modifikationen durch
- Säurebehandlung
- Oxidation
- Veresterung (Enstehung von Phosphat-, Nitrat-, Sulfat-, Xanthat-, Acetat- und Citratstärken. Weitere organische Säuren können ebenfalls zur Veresterung eingesetzt werden.)
- Erzeugung von Stärkeethern (Stärke-Alkylether, O-Allylether, Hydroxylalkylether, O-Carboxylmethylether, N-haltige Stärkeether, S-haltige Stärkeether)
- Erzeugung von vernetzten Stärken
- Erzeugung von Stärke-Pfropf-Polymerisaten.

Gegenstand der Erfindung ist auch Vermehrungsmaterial der erfindungsgemäßen Pflanzen, wie z.B. Samen, Früchte, Stecklinge, Knollen oder Wurzelstöcke, wobei dieses erfindungsgemäße Pflanzenzellen enthält.

Folgende im Rahmen der vorliegenden Erfindung hergestellten und/oder verwendeten Plasmide wurden bei der als internationale Hinterlegungsstelle anerkannten Deutschen Sammlung von Mikroorganismen (DSM) in Braunschweig, Bundesrepublik Deutschland, entsprechend den Anforderungen des Budapester Vertrages für die internationale Anerkennung der Hinterlegung von Mikroorganismen zum Zwecke der Patentierung hinterlegt

| (Hinterlegungsnummer; Hinterlegungsdatum): | | | |
|---|---|---|---|
| Plasmid | pBinAR Hyg | (DSM 9505) | (20.10.1994) |
| Plasmid | p33-anti-BE | (DSM 6146) | (20.08.1990) |
| Plasmid | pRL2 | (DSM 10225) | (04.09.1995) |

| Verwendete Medien und Lösungen | | |
|---|---|---|
| Elutionspuffer | 25 | mM Tris pH 8,3 |
| | 250 | mM Glycin |
| | | |
| Dialysepuffer | 50 | mM Tris-HCl pH 7,0 |
| | 50 | mM NaCl |
| | 2 | mM EDTA |
| | 14,7 | mM β-Mercaptoethanol |
| | 0,5 | mM PMSF |
| | | |
| Proteinpuffer | 50 | mM Natriumphosphatpuffer pH 7,2 |
| | 10 | mM EDTA |
| | 0,5 | mM PMSF |
| | 14,7 | mM β-Mercaptoethanol |
| | | |
| Lugolsche Lösung | 12 g | KI |
| | 6 g | I₂ |
| | ad 1,8 1 | mit ddH₂O |
| 20 x SSC | 175.3 | g NaC1 |
| | 88.2 | g Natrium-Citrat |
| | | ad 1000 ml mit ddH₂O |
| | | pH 7,0 mit 10 N NaOH |
| 10 x MEN | 200 | mM MOPS |
| | 50 | mM Natriumacetat |
| | 10 | mM EDTA |
| | | pH 7, 0 |
| NSEB-Puffer | 0,25 | M Natriumphosphatpuffer pH 7,2 |
| | 7% | SDS |
| | 1 | mM EDTA |
| | 1% | BSA (w/v) |

### Beschreibung der Abbildungen

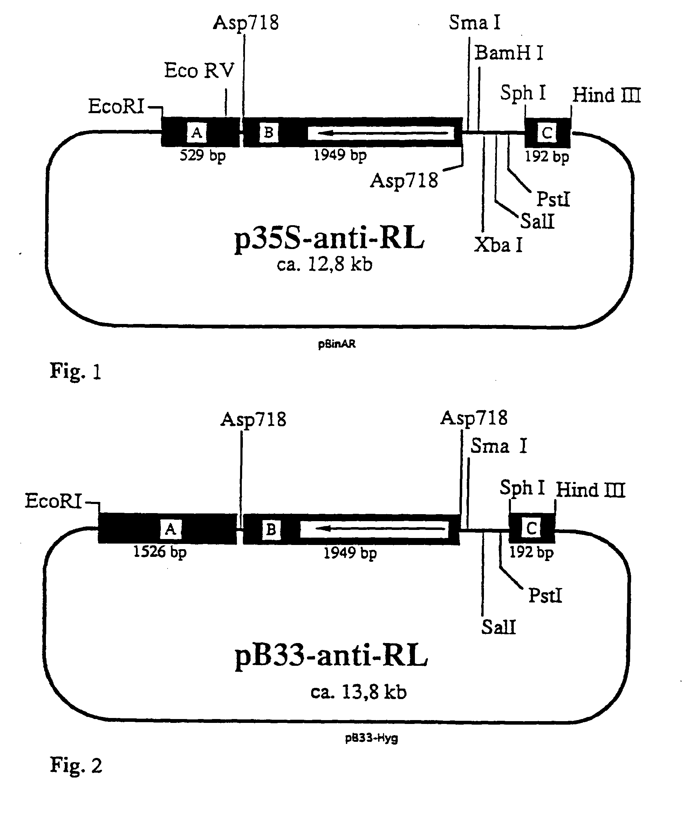

Fig. 1 zeigt das Plasmid p35S-anti-RL.
Aufbau des Plasmids:
A = Fragment A: CaMV 35S-Promotor, nt 6909-7437 (Franck et al., Cell 21 (1980), 285-294)
B = Fragment B: ca. 1949 bp-langes Asp718-Fragment aus **pRL1**
   Orientierung zum Promotor: anti-sense
   Der Pfeil gibt die Richtung des offenen Leserasters an.
C = Fragment C: nt 11748-11939 der T-DNA des Ti-Plasmids pTiACH5 (Gielen et al., EMBO J. 3 (1984), 835-846)
**Fig. 2** zeigt das Plasmid pB33-anti-RL
Aufbau des Plasmids:
A = Fragment A: B33-Promotor des Patatin-Gens B33 aus *Solanum tuberosum* (Rocha-Sosa et al., EMBO J. 8 (1989), 23-29)
B = Fragment B: ca. 1949 bp-langes Asp718-Fragment aus pRL1
   Orientierung zum Promotor: *anti-sense*
   Der Pfeil gibt die Richtung des offenen Leserasters an.
C = Fragment C: nt 11748-11939 der T-DNA des Ti-Plasmids pTiACH5 (Gielen et al., EMBO J. 3 (1984), 835-846)
**Fig. 3** zeigt eine mit einem Brabender-Viskograph vom Typ Viskograph E aufgezeichnete Brabender-Kurve einer wäßrigen Lösung von Stärke, die aus nicht-transformierten Kartoffelpflanzen der Varietät Désirée isoliert wurde (siehe Beispiel 8).

| | | |
|---|---|---|
| Dabei bedeuten: | Drehm. | Drehmoment |
| | [BE] | Brabender-Einheiten |
| | Temp. | Temperatur |
| | A | Verkleisterungsbeginn |
| | B | Maximale Viskosität |
| | C | Start der Haltezeit |
| | D | Start der Kühlzeit |
| | E | Ende der Kühlzeit |
| | F | Ende der End-Haltezeit |

Die blaue Linie gibt die Viskosität an; die rote den Temperaturverlauf.
**Fig. 4** zeigt eine mit einem Brabender-Viskograph vom Typ Viskograph E aufgezeichnete Brabender-Kurve einer wäßrigen Lösung von Stärke, die aus Kartoffelpflanzen isoliert wurde, die mit dem Plasmid p35S-anti-RL transformiert worden waren (siehe Beispiel 8). Für die Bedeutung der Abkürzungen siehe Figur 3.
**Fig. 5** zeigt eine mit einem Brabender-Viskograph vom Typ Viskograph E aufgezeichnete Brabender-Kurve einer wäßrigen Lösung von Stärke aus Kartoffeln, die mit dem Plasmid pB33-anti-RL transformiert worden waren (siehe Beispiel 8). Für die Bedeutung der Abkürzungen siehe Figur 3.
**Fig. 6** zeigt mit einem Rapid Visco Analyser aufgezeichnete Kurven wäßriger Stärkelösungen, die aus Kartoffelpflanzen isoliert wurden (siehe Beispiel 12). Die rote Linie gibt den Temperaturverlauf an, die blauen Linien 1, 2, 3 und 4 die Viskositäten folgender Stärkelösungen:
- Linie 1:: Stärke, die aus Wildtyppflanzen isoliert worden ist,
- Linie 2:: Stärke, die aus Pflanzen isoliert worden ist, bei denen das Verzweigungsenzym alleine inhibiert wurde (vgl. Beispiel 1 der Patentanmeldung WO92/14827),
- Linie 3:: Stärke, die aus Pflanzen isoliert worden ist, bei denen lediglich die erfindungsgemäßen Proteine in ihrer Konzentration verringert wurden (vgl. Beispiel 6).
- Linie 4:: Stärke, die aus Pflanzen isoliert worden ist, die mit dem Plasmid p35S-anti-RL in Kombination mit dem Plasmid p35SH-anti-BE (vgl. Beispiel 12) transformiert worden sind.
**Fig. 7** zeigt mit einem Rapid Visco Analyser aufgezeichnete Kurven wäßriger Stärkelösungen, die aus Kartoffelpflanzen isoliert wurden (siehe Beispiel 13). Die rote Linie gibt den Temperaturverlauf an, die blauen Linien 1, 2, 3 und 4 die Viskositäten folgender Stärkelösungen:
- Linie 1:: Stärke, die aus Wildtyppflanzen isoliert worden ist,
- Linie 2:: Stärke, die aus Pflanzen isoliert worden ist, die **allein mit dem Plasmid pB33-anti-GBSSI isoliert** wurde (sog. waxy-Kartoffel),
- Linie 3:: Stärke, die aus Pflanzen isoliert worden ist, die allein mit dem Plasmid p35S-anti-RL transformiert wurde (vgl. Beispiel 6),
- Linie 4:: Stärke, die aus Pflanzen isoliert worden ist, die mit dem Plasmid pB33-anti-RL in Kombination mit dem Plasmid pB33-anti-GBSSI (vgl. Beispiel 13) transformiert worden sind.

Die Beispiele erläutern die Erfindung.

In den Beispielen wurden folgende Standardtechniken angewendet:
1. Klonierungsverfahren
Zur Klonierung in *E.coli* wurde der Vektor pBluescriptSK verwendet.
Für die Pflanzentransformation wurden die Genkonstruktionen in den binären Vektor pBinAR (Höfgen und Willmitzer, Plant Sci. 66 (1990), 221-230) und B33-Hyg kloniert.
2. Bakterienstämme
Für den pBluescript-Vektor und für die pBinAR- und B33-Hyg-Konstrukte wurde der *E.coli*-Stamm DH5a (Bethesda Research Laboratories, Gaithersburgh, USA) verwendet.
Die Transformation der Plasmide in die Kartoffelpflanzen wurde mit Hilfe des *Agrobacterium tumefaciens*-Stammes C58C1 pGV2260 durchgeführt (Deblaere et al., Nucl. Acids Res. 13 (1985), 4777:4788).
3. Transformation von *Agrobacterium tumefaciens*
Der Transfer der DNA erfolgte durch direkte Transformation nach der Methode von Höfgen & Willmitzer (Nucleic Acids Res. 16 (1988), 9877). Die Plasmid-DNA transformierter Agrobakterien wurde nach der Methode von Birnboim & Doly (Nucleic Acids Res. 7 (1979), 1513-1523) isoliert und nach geeigneter Restriktionsspaltung gelelektrophoretisch analysiert.
4. Transformation von Kartoffeln
Zehn kleine mit dem Skalpell verwundete Blätter einer Kartoffel-Sterilkultur (Solanum tuberosum L.cv. Desiree) wurden in 10 ml MS-Medium (Murashige & Skoog, Physiol. Plant. 15 (1962), 473-497) mit 2% Saccharose gelegt, welches 50 µl einer unter Selektion gewachsenen *Agrobacterium tumefaciens*-Übernachtkultur enthielt. Nach 3-5 minütigem, leichtem Schütteln erfolgte eine weitere Inkubation für 2 Tage im Dunkeln. Daraufhin wurden die Blätter zur Kallusinduktion auf MS-Medium mit 1,6% Glucose, 5 mg/l Naphthylessigsäure, 0,2 mg/l Benzylaminopurin, 250 mg/l Claforan, 50 mg/l Kanamycin bzw. 1 mg/l Hygromycin B, und 0,80% Bacto Agar gelegt. Nach einwöchiger Inkubation bei 25°C und 3000 Lux wurden die Blätter zur Sproßinduktion auf MS-Medium mit 1,6% Glucose, 1,4 mg/l Zeatinribose, 20 mg/l Naphthylessigsäure, 20 mg/l Giberellinsäure, 250 mg/l Claforan, 50 mg/l Kanamycin bzw. 3 mg/l Hygromycin B, und 0,80% Bacto Agar gelegt.
5. Radioaktive Markierung von DNA-Fragmenten
Die radiokative Markierung von DNA-Fragmenten wurde mit Hilfe eines DNA-Random Primer Labelling Kits der Firma Boehringer (Deutschland) nach den Angaben des Herstellers durchgeführt.
6. Northern Blot-Analyse
RNA wurde nach Standardprotokollen aus Blattgewebe von Pflanzen isoliert. 50 µg der RNA wurden auf einem Agarosegel aufgetrennt (1,5% Agarose, 1 x MEN-Puffer, 16,6% Formaldehyd). Das Gel wurde nach dem Gellauf kurz in Wasser gewaschen. Die RNA wurde mit 20 x SSC mittels Kapillarblot auf eine Nylonmembran vom Typ Hybond N (Amersham, UK) transferiert. Die Membran wurde anschließend bei 80°C unter Vakuum für zwei Stunden gebacken. Die Membran wurde in NSEB-Puffer für 2 h bei 68°C prähybridisiert und anschließend in NSEB-Puffer über Nacht bei 68°C in Gegenwart der radioaktiv markierten Probe hybridisiert.
7. Pflanzenhaltung
Kartoffelpflanzen wurden im Gewächshaus unter folgenden Bedingungen gehalten:

| | | |
|---|---|---|
| Lichtperiode | 16 h bei | 25000 Lux und 22°C |
| Dunkelperiode | 8 h bei | 15°C |
| Luftfeuchte | 60% | |

8. Bestimmung des Amylose/Amylopektinverhältnisses in Stärke aus Kartoffelpflanzen
Stärke wurde nach Standardmethoden aus Kartoffelpflanzen isoliert und das Verhältnis Amylose zu Amylopektin nach der von Hovenkamp-Hermelink et al. beschriebenen Methode (Potato Research 31 (1988) 241-246) bestimmt.
9. Bestimmung von Glucose, Fructose und Saccharose
Zur Bestimmung des Glucose-, Fructose- bzw. Saccharosegehalts werden kleine Knollenstücke (Durchmesser ca. 10 mm) von Kartoffelknollen in flüssigem Stickstoff eingefroren und anschließend für 30 min bei 80 °C in 0,5 ml 10 mM HEPES, pH 7,5; 80% (Vol./Vol.) Ethanol extrahiert. Der Überstand, der die löslichen Bestandteile enthält, wird abgenommen und das Volumen bestimmt. Der Überstand wird zur Bestimmung der Menge an löslichen Zuckern verwendet. Die quantitative Bestimmung von löslicher Glucose, Fructose und Saccharose wird in einem Ansatz mit folgender Zusammensetzung durchgeführt:

| | |
|---|---|
| 100,0 mM | Imidazol/HCl, pH 6,9 |
| 1,5 mM | MgCl₂ |
| 0,5 mM | NADP⁺ |
| 1,3 mM | ATP |
| 10-50 µl | Probe |
| 1,0 U | Glucose-6-Phosphatdehydrogenase aus Hefe |

Der Ansatz wird für 5 min bei Raumtemperatur inkubiert. Die Bestimmung der Zucker erfolgt anschließend mittels gängiger photometrischer Methoden durch Messung der Absorption bei 340 nm nach der aufeinanderfolgenden Zugabe von

| | |
|---|---|
| 1,0 Einheiten | Hexokinase aus Hefe (zur Bestimmung von Glucose) |
| 1,0 Einheiten | Phosphoglucoisomerase aus Hefe (zur Bestimmung von Fructose) und |
| 1,0 Einheiten | Invertase aus Hefe (zur Bestimmung von Saccharose). |

### Beispiel 1

### Isolierung Stärkekorn-gebundener Proteine aus Kartoffelstärke

Die Isolierung von Stärkekorn-gebundenen Proteinen aus Kartoffelstärke erfolgte durch Elektroelution in einer Elutionsvorrichtung, die analog zu dem "Model 422 Electro-Eluter" (BIORAD Laboratories Inc., USA) konstruiert war, aber ein wesentlich größeres Volumen aufwies (ca. 200 ml). Es wurden 25 g getrocknete Stärke in Elutionspuffer aufgenommen (Endvolumen 80 ml). Die Stärke stammte aus Kartoffeln, die aufgrund der anti-sense-Expression einer DNA-Sequenz, die für die Stärkekorn-gebundene Stärkesynthase I (GBSS I) aus Kartoffel kodiert, eine nahezu amylosefreie Stärke produzieren. Die Suspension wurde im Wasserbad auf 70-80°C erwärmt. Anschließend wurden 72,07 g Harnstoff zugegeben (Endkonzentration 8 M) und das Volumen mit Elutionspuffer auf 180 ml aufgefüllt. Die Stärke löste sich unter ständigem Rühren und bekam eine kleisterartige Konsistenz. Die Proteine wurden aus der Lösung mit Hilfe des Elutionsvorrichtung über Nacht elektroeluiert (100 V; 50-60 mA). Die eluierten Proteine wurden vorsichtig aus der Appartur entnommen. Schwebstoffe wurden durch kurze Zentrifugation entfernt. Der Überstand wurde 2-3 mal je eine Stunde bei 4°C gegen Dialysepuffer dialysiert. Anschließend wurde das Volumen der Proteinlösung bestimmt. Die Proteine wurden durch Zugabe von Ammoniumsulfat (90% Endkonzentration) gefällt. Die Zugabe erfolgte unter ständigem Rühren bei 0°C. Die gefällten Proteine wurden durch Zentrifugation pelletiert und in Proteinpuffer aufgenommen.

### Beispiel 2

### Identifizierung und Isolierung von cDNA-Sequenzen, die für Stärkekorn-gebundene Proteine kodieren

Die gemäß Beispiel 1 isolierten Proteine wurden zur Herstellung von polyclonalen Antikörpern aus Kaninchen verwendet, die spezifisch Stärkekorn-gebundene Proteine erkennen. Mit Hilfe derartiger Antikörper wurde anschließend nach Standardmethoden eine cDNA-Expressionsbibliothek nach Sequenzen durchgemustert, die für Stärkekorn-gebundene Proteine kodieren.
Die Expressionsbibliothek wurde folgendermaßen hergestellt: Aus Kartoffelknollen der Kartoffelvarietät "Berolina" wurde nach Standardmethoden poly(A⁺)-mRNA isoliert. Ausgehend von der poly(A⁺)-mRNA wurde nach der Methode von Gubler und Hoffmann (Gene 25 (1983), 263-269) unter Verwendung eines *Xho* I-Oligo d(t)₁₈-Primers cDNA hergestellt. Diese wurde nach EcoR I-Linkeraddition mit *Xho* I nachgeschnitten und orientiert in einen mit EcoR I und *Xho* I geschnittenen Lambda ZAP II-Vektor (Stratagene) ligiert. Ca. 500.000 Plaques einer derart konstruierten cDNA-Bibliothek wurden nach Sequenzen durchgemustert, die von polyclonalen Antikörpern, die gegen Stärkekorn-gebundene Proteine gerichtet sind, erkannt wurden.
Zur Analyse der Phagenplaques wurden diese auf Nitrozellulosefilter übertragen, die vorher für 30-60 min in einer 10 mM IPTG-Lösung inkubiert und anschließend auf Filterpapier getrocknet wurden. Der Transfer erfolgte für 3 h bei 37°C. Anschließend werden die Filter für 30 min bei Raumtemperatur in Blockreagenz inkubiert und zweimal für 5-10 min in TBST-Puffer gewaschen. Die Filter wurden mit den gegen Stärkekorn-gebundene Proteine gerichteten polyclonalen Antikörpern in geeigneter Verdünnung für 1 h bei Raumtemperatur oder für 16 h bei 4°C geschüttelt. Die Identifizierung von Plaques, die ein Protein exprimierten, das von den polyclonalen Antikörpern erkannt wurde, erfolgte mit Hilfe des "Blotting detection kit for rabbit antibodies RPN 23" (Amersham UK) nach den Angaben des Herstellers.
Phagenclone der cDNA-Bibliothek, die ein Protein exprimierten, das von den polyclonalen Antikörpern erkannt wurde, wurden unter Anwendung von Standardverfahren weiter gereinigt.
Mit Hilfe der in-vivo-excision-Methode wurden von positiven Phagenclonen E. coli-Klone gewonnen, die ein doppelsträngiges pBluescript-Plasmid mit der jeweiligen cDNA-Insertion enthalten. Nach Überprüfung der Größe und des Restriktionsmusters der Insertionen wurde ein geeigneter Clon, pRL1, weiter analysiert.

### Beispiel 3

### Sequenzanalyse der cDNA-Insertion des Plasmids pRL1

Aus einem entsprechend Beispiel 2 erhaltenen *E. coli*-Clon wurde das Plasmid pRL1 isoliert und ein Teil der Sequenz seiner cDNA-Insertion durch Standardverfahren mittels der Didesoxynukleotidmethode (Sanger et al., Proc. Natl. Acad. Sci. USA 74 (1977), 5463-5467) bestimmt. Die Insertion ist ca. 2450 bp lang. Ein Teil der Nukleotidsequenz sowie die daraus abgeleitete Aminosäuresequenz ist unter Seq ID No. 3 und Seq ID No. 4 angegeben.
Eine Sequenzanalyse und ein Sequenzvergleich mit bekannten DNA-Sequenzen zeigte, daß die unter Seq ID No. 3 dargestellte Sequenz neu ist und keine signifikante Homologie zu bisher bekannten DNA-Sequenzen aufweist. Die Sequenzanalyse zeigte weiterhin, daß es sich bei der cDNA-Insertion nur um eine partielle cDNA handelt, bei der ein Teil der codierenden Region am 5'-Ende fehlt.

### Beispiel 4

### Identifizierung und Isolierung einer vollständigen cDNA, die für ein Stärkekorn-gebundenes Protein aus Solanum tuberosum codiert

Zur Isolierung einer, der partiellen cDNA-Insertion des Plasmids pRL1 entsprechenden, vollständigen cDNA, wurde eine weitere cDNA-Bibliothek hergestellt. Dabei handelte es sich um eine Schließzellen-spezifische cDNA-Bibliothek aus Solanum *tuberosum,* die folgendermaßen konstruiert wurde: Zunächst wurden Epidermisfragmente von Blättern von Kartoffelpflanzen der Varietät Desiree im wesentlichen nach der Methode von Hedrich et al. (Plant Physiol. 89 (1989), 148) hergestellt indem ca. 60 Blätter von sechs Wochen alten, im Gewächshaus gehaltenen Kartoffelpflanzen geerntet wurden. Aus den Blättern wurde die Mittelrippe entfernt. Anschließend wurden die Blätter in einem großen "Waring blender" (Volumen 1 Liter) in gekühltem destilliertem H₂O viermal für je 15 Sekunden auf höchster Stufe zerkleinert. Die Suspension wurde durch ein Nylonsieb mit einer Porengröße von 220 µm (Nybolt, Zürich, Schweiz) filtriert und mehrmals mit kaltem destilliertem Wasser gewaschen. Die Suspension wurde wiederum durch ein 220 µm-Nylonsieb filtriert und ausgiebig mit kaltem destilliertem Wasser gewaschen. Der Rückstand (Epidermisfragmente) wurde in einen kleineren "Waring blender" (Volumen 250 ml) gegeben und in destilliertem Wasser und Eis viermal für je 15 Sekunden bei auf kleiner Stufe zerkleinert. Die Suspension wurde durch ein 220 µm-Nylonsieb filtriert und ausgiebig mit kaltem destilliertem Wasser gewaschen. Die Epidermisfragmente (Rückstand) wurden mikroskopisch hinsichtlich einer Kontamination durch Mesophyllzellen untersucht. Wenn dies der Fall war, wurde der Zerkleinerungsschritt im kleinen "Waring blender" wiederholt.
Der Aufschluß der Schließzellen der Epidermisfragmente erfolgte durch Zermörsern in flüssigem Stickstoff in einem gekühlten Mörser für ca. 2 h. Zur Überprüfung des Aufschlusses der Schließzellen wurden regelmäßig Proben genommen und mikroskopisch überprüft. Nach 2 h oder wenn eine genügend große Anzahl von Schließzellen aufgeschlossen wurde, wurde das entstandene Pulver in ein Reaktionsgefäß (Volumen 50 ml) überführt und in einem Volumen GTC-Puffer (Chirgwin et al., Biochem. 18 (1979), 5294-5299) aufgenommen. Die Suspension wurde zentrifugiert und der Überstand durch Miracloth (Calbiochem, La Jolla, Kalifornien) filtriert. Das Filtrat wurde wie in Glisin et al. (Biochemistry 13 (1974), 2633-2637) und Mornex et al. (J. Clin. Inves. 77 (1986), 1952-1961) für 16 h einer Ultrazentrifugation unterzogen. Nach der Zentrifugation wurde der RNA-Niederschlag in 250 µl GTC-Puffer aufgenommen. Die RNA wurde durch Zugabe von 0,05 Volumen 1 M Essigsäure und 0,7 Volumen Ethanol gefällt. Die RNA wurde abzentrifugiert und der Niederschlag mit 3 M Natriumazetat (pH 4,8) und 70% Ethanol gewaschen. Die RNA wurde kurz getrocknet und in DEPC-behandeltem Wasser gelöst.
Aus der isolierten RNA wurde nach Standardverfahren poly A⁺⁻RNA isoliert. Ausgehend von der poly(A⁺)-mRNA wurde nach der Methode von Gubler und Hoffmann (Gene 25 (1983), 263-269) unter Verwendung eines *Xho* I-Oligo d(t)₁₈-Primers cDNA hergestellt. Diese wurde nach *Eco*R I-Linkeraddition mit *Xho* I nachgeschnitten und orientiert in einen mit *Eco*R I und *Xho* I geschnittenen Lambda ZAP II-Vektor (Stratagene, GmbH, Heidelberg, Deutschland) ligiert. Das Verpacken in Phagenköpfe erfolgte unter Verwendung des Gigapack II Gold kit's (Stratagene, GmbH, Heidelberg, Deutschland) nach den Angaben des Herstellers.
Aus einer derartigen cDNA-Bibliothek wurden nach Standardverfahren Phagenclone isoliert und gereinigt, die mit der cDNA-Insertion aus dem Plasmid pRL1 hybridisieren. Mit Hilfe der in-vivo-excision-Methode wurden von positiven Phagenclonen *E. coli*-Klone gewonnen, die ein doppelsträngiges pBluescript-Plasmid mit der jeweiligen cDNA-Insertion enthalten. Nach Überprüfung der Größe und des Restriktionsmusters der Insertionen wurden geeignete Klone einer Restriktionskartierung und einer Sequenzanalyse unterzogen. Aus einem geeigneten Clon wurde das Plasmid pRL2 (DSM 10225) isoliert, das eine vollständige cDNA enthält, die für ein Stärkekorn-gebundenes Protein aus Kartoffel codiert.

### Beispiel 5

### Sequenzanalyse der cDNA-Insertion des Plasmids pRL2

Die Nukleotidsequenz der cDNA-Insertion des Plasmids pRL2 wurde wie in Beispiel 3 beschrieben bestimmt. Die Insertion ist 4856 bp lang. Die Nukleotidsequenz sowie die daraus abgeleitete Aminosäuresequenz ist in Seq ID No. 1 bzw. Seq ID No. 2 angegeben. Das entsprechende Gen wird im folgenden RL-Gen genannt.

### Beispiel 6

### Konstruktion des Plasmids p35S-anti-RL und Einführung des Plasmids in das Genom von Kartoffelpflanzen

Aus dem Plasmid pRL1 wurde mit Hilfe der Restriktionsendonuklease Asp718 ein ca. 1800 bp langes DNA-Fragment isoliert. Dieses entspricht der unter Seq ID No. 3 dargestellten DNA-Sequenz und enthält einen Teil des offenen Leserahmens. Das Fragment wurde in den mit Asp718 geschnittenen binären Vektor pBinAR (Höfgen und willmitzer, Plant Sci. 66 (1990), 221-230) ligiert. Bei diesem handelt es sich um ein Derivat des binären Vektors pBin19 (Bevan, Nucl. Acids Res. 12 (1984), 8711-8721). pBinAR wurde folgendermaßen konstruiert:

Ein 529 bp langes Fragment, das die Nukleotide 6909-7437 des 35S-Promotor des Cauliflowermosaic-Virus umfaßt (Franck et al., Cell 21 (1980), 285-294), wurde als *EcoR* I/*Kpn I-*Fragment aus dem Plasmid pDH51 (Pietrzak et al., Nucl. Acids Res. 14, 5857-5868) isoliert und zwischen die EcoR I- und die Kpn I-Schnittstellen des Polylinkers von pBinl9 ligiert. Dabei entstand das Plasmid pBin19-A.
Aus dem Plasmid pAGV40 (Herrera-Estrella et al., Nature 303, 209-213) wurde mit Hilfe der Restriktionsendonukleasen Pvu II und *Hind* III ein 192 bp langes Fragment isoliert, das das Polyadenylierungssignal des Gens 3 der T-DNA des Ti-Plasmids pTiACH5 (Gielen et al., EMBO J. 3, 835-846) umfaßt (Nukleotide 11749-11939). Nach Addition von Sph I-Linkern an die Pvu I-Schnittstelle wurde das Fragment zwischen die *Sph* I- und *Hind* III-Schnittstellen pBin19-A ligiert. Dabei entstand pBinAR.
Mit Hilfe von Restriktions- und Sequenzanalysen wurden rekombinante Vektoren identifiziert, bei denen das DNA-Fragment derart in den Vektor inseriert ist, daß ein Teil der codierenden Region der cDNA-Insertion aus pRL1 in *anti-sense*-Orientierung mit dem 35S-Promotor verknüpft ist. Das resultierende Plasmid, p35S-anti-RL, ist in Fig. 1 dargestellt.
Durch die Insertion des cDNA-Fragmentes entsteht eine Expressionskassette, die folgendermaßen aus den Fragmenten A, B und C aufgebaut ist:
Das Fragment A (529 bp) enthält den 35S-Promotor des Cauliflower-Mosaik-Virus (CaMV). Das Fragment umfaßt die Nukleotide 6909 bis 7437 des CaMV (Franck et al., Cell 21 (1980), 285-294).
Das Fragment B enthält neben flankierenden Bereichen einen Teil der proteincodierenden Region der cDNA-Insertion aus dem Plasmid pRL1. Diese wurde wie oben beschrieben als Asp718-Fragment aus pRL1 isoliert und in anti-sense-Orientierung an den 35S-Promotor in pBinAR fusioniert.

Fragment C (192 bp) enthält das Polyadenylierungssignal des Gens 3 der T-DNA des Ti-Plasmids pTiACH5 (Gielen et al., EMBO J. 3 (1984), 835-846).
Die Größe des Plasmids p35S-anti-RL beträgt ca. 12,8 kb.
Das Plasmid wurde mit Hilfe Agrobakterien-vermittelter Transformation in Kartoffelpflanzen transferiert wie oben beschrieben. Aus den transformierten Zellen wurden ganze Pflanzen regeneriert. Die transformierten Pflanzen wurden unter Gewächshausbedingungen kultiviert.
Die Überprüfung des Erfolges der genetischen Veränderung der Pflanzen erfolgte durch Analyse der Gesamt-RNA in einer Northern-Blot-Analyse bezüglich des Verschwindens der zu der cDNA komplementären Transkripte. Hierzu wurde Gesamt-RNA aus Blättern transformierter Pflanzen nach Standardmethoden isoliert, gelelektrophoretisch auf einem Agarosegel aufgetrennt, auf eine Nylonmembran transferiert und mit einer radioaktiv markierten Probe hybridisiert, die die unter Seq ID No. 1 dargestellte Sequenz oder einen Teil dieser Sequenz aufweist. In ca. 5-10% der transformierten Pflanzen fehlte in der Northern-Blot-Analyse die Bande, die das spezifische Transkript der unter Seq ID No. 1 dargestellten Sequenz darstellt. Diese Pflanzen wurden zur Analyse der Stärkequalität verwendet.

### Beispiel 7

### Konstruktion des Plasmids pB33-anti-RL und Einführung des Plasmids in das Genom von Kartoffelpflanzen

Aus dem Plasmid pRL1 wurde mit Hilfe der Restriktionsendonuklease Asp718 ein ca. 1800 bp langes DNA-Fragment isoliert, das einen Teil des offenen Leserahmens der cDNA-Insertion umfaßt, und in den mit *Asp*718 geschnittenen Vektor B33-Hyg ligiert. Dieser Vektor wurde folgendermaßen hergestellt: Aus dem Vektor pBinAR Hyg (DSM 9505) wurde mit Hilfe der Restriktionsendonukleasen EcoR I und Asp718 der 35S-Promotor entfernt. Aus dem Plasmid p33-anti-BE (DSM 6146) wurde mit Hilfe von EcoR I und Asp718 ein ca. 1526 bp langes Fragment, das den B33-Promotor umfaßt, isoliert und in den mit EcoR I und Asp718 geschnittenen Vektor pBinAR Hyg (DSM 9505) inseriert.
Durch die Insertion des cDNA-Fragmentes in die *Asp*718-Schnittstelle des Plasmids B33-Hyg entsteht eine Expressionskassette, die folgendermaßen aus den Fragmenten A, B und C aufgebaut ist (Fig. 4):
Das Fragment A enthält den B33-Promotor aus *Solanum tuberosum* (EP 3775 092; Rocha-Sosa et al., EMBO J. 8 (1989), 23-29)
Das Fragment B enthält neben flankierenden Bereichen einen Teil der proteincodierenden Region der cDNA-Insertion aus dem Plasmid pRL1. Diese wurde wie oben beschrieben als *Asp*718-Fragment aus pRL1 isoliert und in *anti-sense-*Orientierung an den B33-Promotor in B33-Hyg fusioniert. Fragment C (192 bp) enthält das Polyadenylierungssignal des Gens 3 der T-DNA des Ti-Plasmids pTiACH5 (Gielen et al., EMBO J. 3 (1984), 835-846).
Die Größe des Plasmids pB33-anti-RL beträgt ca. 12,8 kb.
Das Plasmid wurde mit Hilfe Agrobakterien-vermittelter Transformation in Kartoffelpflanzen transferiert wie oben beschrieben. Aus den transformierten Zellen wurden ganze Pflanzen regeneriert. Die transformierten Pflanzen wurden unter Gewächshausbedingungen kultiviert.
Die Überprüfung des Erfolges der genetischen Veränderung der Pflanzen erfolgte durch Analyse der Gesamt-RNA in einer Northern-Blot-Analyse bezüglich des Verschwindens der zu der cDNA komplementären Transkripte. Hierzu wurde Gesamt-RNA aus Knollen transformierter Pflanzen nach Standardmethoden isoliert, gelelektrophoretisch auf einem Agarosegel aufgetrennt, auf eine Nylonmembran transferiert und mit einer radioaktiv markierten Probe hybridisiert, die die unter Seq ID No. 1 dargestellte Sequenz oder einen Teil dieser Sequenz aufweist. In ca. 5-10% der transformierten Pflanzen fehlte in der Northern-Blot-Analyse die Bande, die Transkripte darstellt, die mit der erfindungsgemäßen cDNA hybridisieren. Aus diesen Pflanzen wurde aus Knollen die Stärke isoliert und wie in Beispiel 8 beschrieben analysiert.

### Beispiel 8

### Analyse der transformierten Kartoffelpflanzen

Die gemäß Beispiel 6 und Beispiel 7 transformierten Kartoffelpflanzen wurden hinsichtlich der Eigenschaften der synthetisierten Stärke untersucht. Die Analysen wurden an verschiedenen Linien von Kartoffelpflanzen durchgeführt, die mit dem Plasmid p35S-anti-RL bzw. mit dem Plasmid pB33-anti-RL transformiert worden waren und die in der Northern-Blot-Analyse die Bande nicht mehr aufwiesen, die Transkripte darstellt, die mit den erfindungsgemäßen DNA-Sequenzen hybridisieren.

### a) Bestimmung der Viskosität wäßriger Lösungen der Stärke

Zur Bestimmung der Viskosität der wäßrigen Lösungen der in transformierten Kartoffelpflanzen synthetisierten Stärke wurde aus Knollen von Pflanzen, die mit dem Plasmid p35S-anti-RL bzw. mit dem Plasmid pB33-anti-RL transformiert worden waren, Stärke nach Standardverfahren isoliert. Es wurden jeweils 30 g Stärke in 450 ml H₂O aufgenommen und für die Analyse in einem Viskograph E (Brabender OHG Duisburg (Deutschland)) verwendet. Der Betrieb des Gerätes erfolgte nach den Angaben des Herstellers. Zur Bestimmung der Viskosität der wäßrigen Lösung der Stärke wurde die Stärkesuspension zunächst von 50°C auf 96°C erhitzt mit einer Geschwindigkeit von 3°C pro min. Anschließend wurde die Temperatur für 30 min bei 96°C gehalten. Danach wurde die Lösung von 96°C auf 50°C abgekühlt mit einer Geschwindigkeit von 3°C pro min. Während der gesamten Dauer wurde die Viskosität bestimmt. Repräsentative Ergebnisse derartiger Messungen sind in Form von Kurven, in denen die Viskosität in Abhängigkeit der Zeit dargestellt ist, in Fig. 3, Fig. 4 und Fig. 5 wiedergegeben. Fig. 3 zeigt eine typische Brabenderkurve für Stärke, die aus Wildtyp-Pflanzen der Kartoffelvarietät Désirée isoliert wurde. Fig. 4 und 5 zeigen eine typische Brabenderkurve für Stärke, die aus Kartoffelpflanzen isoliert wurde, die mit dem Plasmid p35S-anti-RL bzw. pB33-anti-RL transformiert worden waren. Aus den Kurven lassen sich verschiedene charakteristische Werte ableiten.

Für Wildtyppflanzen ergeben sich dabei folgende charakteristische Werte:

**Tabelle 1**

| Wert | Zeit [min : sec] | Drehmoment [BE] | Temperatur [°C] |
|---|---|---|---|
| A | 6 : 30 | 60,5 ± 17,7 | 69,9 ± 0,57 |
| B | 11 : 30 | 1838,0 ± 161,2 | 86,0 ± 2,1 |
| C | 15 : 15 | 1412,0 ± 18,4 | 96,0 |
| D | 45 : 15 | 526,0 ± 17,0 | 96,0 |
| E | 60 : 30 | 812,0 ± 8,5 | 50,0 |
| F | 70 : 45 | 853,0 ± 5,7 | 50,0 |

Die Werte geben Mittelwerte aus zwei verschiedenen Messungen wieder.

In der Tabelle 1 und den folgenden Tabellen 2 und 3 bedeuten:
- A:: Verkleisterungsbeginn
- B:: Maximale Viskosität
- C: Start der Haltezeit
- D:: Start der Kühlzeit
- E:: Ende der Kühlzeit
- F:: Ende der End-Haltezeit.

Für Pflanzen, die mit dem Plasmid p35S-anti-RL transformiert worden waren (Linie P2), ergeben sich dabei folgende charakteristische Werte:

**Tabelle 2**

| Wert | Zeit (min : sec] | Drehmoment [BE] | Temperatur [°C] |
|---|---|---|---|
| A | 6 : 00 | 50,0 | 69,0 |
| B | 14 : 00 | 820,0 | 93,0 |
| C | 15 : 15 | 815,0 | 96,0 |
| D | 45 : 15 | 680,0 | 96,0 |
| E | 60 : 30 | 1150,0 | 50,0 |
| F | 70 : 45 | 1200,0 | 50,0 |

Für Pflanzen, die mit dem Plasmid pB33-anti-RL transformiert worden waren (Linie P3), ergeben sich dabei folgende Werte:

**Tabelle 3**

| Wert | Zeit [min : sec] | Drehmoment [BE] | Temperatur [°C] |
|---|---|---|---|
| A | 7:0 | 31,0 | 71,0 |
| B | 12:45 | 671,0 | 88,3 |
| C | 15:15 | 662,0 | 96,0 |
| D | 45:15 | 607,0 | 96,0 |
| E | 60:30 | 1063,0 | 50,0 |
| F | 70:45 | 1021,0 | 50,0 |

Aus den Figuren 3, 4 und 5 geht deutlich hervor, daß die Stärke aus transformierten Pflanzen sich von der aus Wildtyp-Pflanzen insbesondere dadurch unterscheidet, daß beim Aufkochen nur eine sehr geringe Viskositätszunahme erfolgt. So liegt die maximale Viskosität bei der modifizierten Stärke aus transformierten Pflanzen beim Auf**kochen um mehr als 50% unter dem Wert der Wildtyp-**Stärke. Andererseits steigt die Viskosität der aus transformierten Pflanzen isolierten Stärke nach dem Abkühlen stärker an als bei Wildtyp-Stärke.

### b) Bestimmung des Phosphatgehaltes der Stärke

Der Phosphatgehalt der Stärke wurde bestimmt, indem die Menge an Phosphat, das an der C-6-Position von Glucoseresten gebunden war, gemessen wurde. Hierzu wurde Stärke zunächst durch Säurehydrolyse gespalten und anschließend der Gehalt an Glucose-6-Phosphat mittels eines Enzymtests bestimmt, wie im folgenden beschrieben.

100 mg Stärke wurden in 500 µl 0,7 N HCl 4 h bei 100 °C inkubiert. Nach der Säurehydrolyse wurden 10 µl des Ansatzes in 600 µl Imidazolpuffer (100 mM Imidazol, 5 mM MgCl₂, pH 6,9, 0,4 mM NAD⁺) gegeben. Die Bestimmung der Menge an Glucose-6-Phosphat in dem Ansatz erfolgte durch Umsetzung mit dem Enzym Glucose-6-Phosphat-Dehydrogenase. Dazu wurde dem Ansatz 1 U Glucose-6-Phosphat-Dehydrogenase (aus Leuconostoc mesenteroides (Boehringer Mannheim)) zugesetzt und die Menge an gebildetem NADH durch Messung der Absorption bei 340 nm bestimmt.

Der Gehalt an Glucose-6-Phosphat/Milligramm Stärke ist in der folgenden Tabelle für nicht-transformierte Kartoffelpflanzen der Varietät Désirée sowie für zwei Linien (P1(35S-anti-RL; P2(35S-anti-RL)) transgener Kartoffelpflanzen, die mit dem Plasmid p35S-anti-RL transformiert worden waren, angegeben.

**Tabelle 4**

| Pflanzen | nmol Glucose-6-Phosphat/mg Stärke | % |
|---|---|---|
| Wildtyp | 12,89 ± 1,34 | 100 |
| P1(35S-anti-RL) | 2,25 ± 0,41 | 17,4 |
| P2(35S-anti-RL) | 1,25 ± 0 | 9,7 |

Die folgende Tabelle zeigt den Glucose-6-Phosphat-Gehalt pro Milligramm Stärke bei Kartoffelpflanzen, die mit dem Plasmid pB33-anti-RL transformiert worden waren, im Vergleich zu Stärke aus nicht-transformierten Pflanzen (S. tuberosum c.v. Désirée).

**Tabelle 5**

| Pflanzen | nmol Glucose-6-Phosphat/mg Stärke | % |
|---|---|---|
| Wildtyp | 9,80 ± 0,68 | 100 |
| 7 | 4,50 ± 0,73 | 45,9 |
| 37 | 2,64 ± 0,99 | 26,9 |
| 45 | 1,14 ± 0,44 | 11,6 |
| 31 | 1,25 ± 0,49 | 12,8 |

Die Pflanzen 7, 37, 45 und 31 stellen unabhängige Transformanten dar, die mit dem Plasmid pB33-anti-RL transformiert worden waren. Die Pflanze 37 repräsentiert die Linie P3, für die in Figur 5 eine Brabenderkurve dargestellt ist.

Die Werte zeigen, daß der Phosphatgehalt der modifizierten Stärke aus transgenen Kartoffelpflanzen um mindestens ca. 50% im Vergleich zu Stärke aus Wildtyp-Pflanzen verringert ist.

### c) Bestimmung des Glucose-, Fructose- und Sacäharosegehalts von Knollen nach Lagerung bei 4 °C

Knollen von Pflanzen verschiedener transgener Linien, die mit dem antisense-Konstrukt p35S-anti-RL transformiert worden waren, und von Wildtyp-Pflanzen wurden für 2 Monate bei 4 °C bzw. bei 20 °C im Dunkeln gelagert. Anschließend wurden wie oben beschrieben die Mengen an Glucose, Fructose und Saccharose bestimmt. Dabei ergaben sich für zwei transgene Linien folgende repräsentative Werte:

**Tabelle 6**

| | Glucose | | Fructose | | Saccharose | |
|---|---|---|---|---|---|---|
| | 20°C | 4°C | 20°C | 4°C | 20°C | 4°C |
| Wildtyp cv Désirée | 0,84 | 55,4 | 0,62 | 52,8 | 8,5 | 13,1 |
| Transgene Linie 15 | 1,12 | 6,7 | 0,75 | 7,8 | 7,5 | 10,1 |
| Transgene Linie 11 | 1,00 | 6,4 | 0,75 | 7,5 | 6,9 | 6,9 |

Die Werte in der Tabelle sind in µmol Hexose bzw. Saccharose/ g Frischgewicht angegeben.

Aus den Werten in Tabelle 6 wird deutlich, daß bei den transgenen Pflanzen bei einer Lagerung bei 4 °C eine wesentlich geringere Akkumulation reduzierender Zucker in den Knollen stattfindet als bei Wildtyp-Pflanzen.

Insgesamt ähnelt die aus transgenen Kartoffelpflanzen isolierte modifizierte Stärke der Stärke aus Mais-Wildtyp-Pflanzen. Im Vergleich zu dieser besitzt sie den Vorteil, daß sie geschmacksneutral ist und so für verschiedene Verwendungsmöglichkeiten im Nahrungsmittelbereich besser geeignet ist.

### Beispiel 9

### Expression der cDNA-Insertion des Plasmids pRL2 in E. coli

### (a) Transformation von Bakterienzellen

Zur Expression der cDNA-Insertion des Plasmids pRL2 wurden Zellen des E. coli-Stammes DH5α zunächst mit dem Plasmid pACAC transformiert. Dieses Plasmid enthält ein DNA-Fragment, das die ADP-Glucose-Pyrophosphorylase (AGPase) aus E. coli codiert, unter der Kontrolle des lac Z-Promotors. Das Fragment war als ca. 1,7 kb großes DraI/HaeII-Fragment aus dem Vektor pEcA-15 (siehe B. Müller-Röber (1992), Dissertation, FU Berlin) isoliert worden und nach Glättung der Enden in einen mit HindIII linearisierten pACAC184-Vektor cloniert worden. Die Expression der AGPase soll eine Steigerung der Glycogensynthese in transformierten E. coli Zellen bewirken. Die derart transformierten Zellen werden im folgenden als E. coli-Kl-Zellen bezeichnet.
Zur Bestimmung der Enzymaktivität des durch die cDNA des Plasmids pRL2 codierten Proteins, wurden E. coli-K1 Zellen mit dem Plasmid pRL2 transformiert. Die transformierten E. coli-Zellen, die sowohl das Plasmid pACAC als auch das Plasmid pRL2 enthalten, werden im folgenden als E. coli-K2-Zellen bezeichnet.
Der Transfer der Plasmid-DNA in die Bakterienzellen erfolgte jeweils nach der Methode von Hanahan (J. Mol. Biol. 166 (1983), 557-580). Die transformierten E. coli Zellen wurden auf Agarkulturschalen mit folgender Zusammensetzung ausgestrichen:
YT-Medium mit

| | |
|---|---|
| 1,5% | Bacto-Agar |
| 50 mM | Natriumphosphat-Puffer, pH 7,2 |
| 1% | Glucose |
| 10 µg/ml | Chloramphenicol bei E. coli-K1-Zellen |
| bzw. | |
| 10 µg/ml | Chloramphenicol und |
| 10 µg/ml | Ampicillin bei E. coli-K2-Zellen. |

Escherichia coli Zellen des Stammes DH5_, die mit dem Plasmid pRL2 + pACAC (E. coli-K2-Zellen) sowie als Kontrolle nur mit dem Plasmid pACAC (E. coli-Kl-Zellen) transformiert worden sind, wurden auf Agarplatten angezogen. Das gebildete Glycogen der verschiedenen Kulturen wurde bezüglich des Phosphorylierungsgrades (an C-6-Position des Glucosemoleküls) hin untersucht, wie im folgenden beschrieben wird.

### (b) Isolierung von bakteriellem Glycogen

Zur Isolierung von bakteriellem Glycogen wurde der nach der Transformation gewachsene Bakterienrasen von jeweils 6 Agarplatten (0 135 mm) mit 5 ml YT-Medium/Platte abgeschwemmt. Die Bakteriensuspension wurde bei 4500 xg für 5 Minuten zentrifugiert. Der Bakterienniederschlag wurde in 10 ml YT-Medium resuspendiert. Der Aufschluß der Bakterien erfolgte durch Zugabe von 2 Volumen Aufschlußmedium (0,2 N NaOH; 1% SDS) und Inkubation für 5 Minuten bei RT. Durch Zugabe von 3 Volumen EtOH abs., 30 minütiger Inkubation bei 4°C und anschließender Zentrifugation von 15 Minuten bei 8000 gx wurde das Glycogen sedimentiert. Anschließend wurde der Niederschlag mit 100 ml 70%igem EtOH gewaschen und erneut durch einen Zentrifugationsschritt (10 Minuten bei 8000 xg) sedimentiert. Der Waschvorgang wurde 4 mal wiederholt.

### (c) Bestimmung des Gesamtglycogengehaltes

Das isolierte und sedimentierte Glycogen wurde zunächst durch saure Hydrolyse (Lösen des Niederschlags in 2 ml 0,7 N HCl; Inkubation für 4 Stunden bei 100°C) in die einzelnen Glucosemoleküle aufgespalten. Der Glucosegehalt der Lösung wurde mittels gekoppelter enzymatischer Reaktion eines Stärke-Tests nach Angaben des Herstellers (Boehringer Mannheim) an einem Photometer (Firma Kontron) bei einer Wellenlänge von 340 nm bestimmt. Der Reaktionspuffer enthält:

| | |
|---|---|
| 100 | mM MOPS, pH 7,5 |
| 10 | mM MgCl₂ |
| 2 | mM EDTA |
| 0,25 | mM NADP |
| 1 | mM ATP |
| 1 | U/ml Glucose-6-Phosphat-Dehydrogenase |
| 2 | U/ml Hexokinase |

Die Messung erfolgte bei 25°C mit 10 µl Glucoselösung.

### (d) Bestimmung des Glucose-6-Phosphat Gehaltes

Zur Bestimmung des Gehaltes der an C-6-Position phosphorylierten Glucosemoleküle wurden gleiche Stoffmengen an Glucose, jeweils der verschiedenen Bakterienkulturen, eingesetzt. Durch Zugabe von gleichen Volumina an 0,7 N KOH zu dem mittels saurer Hydrolyse (siehe oben) in seine Glucosemoleküle aufgespaltenen Glycogens, wurde die Lösung neutralisiert. Der Reaktionspuffer enthält:

| | |
|---|---|
| 100 | mM MOPS, pH 7,5 |
| 10 | mM MgCl₂ |
| 2 | mM EDTA |
| 0,25 | mM NADP |
| 2 | U/ml Glucose-6-Phosphat-Dehydrogenase |

Die Messung erfolgte bei 25°C mit 100-150 µl Glucoselösung.

### (e) Nachweis einer bakterielles Glvcoaen phosphorylierenden Enzymaktivität

Die Ergebnisse der Bestimmung des Phosphatgehaltes des in den Bakterienzellen synthetisierten Glycogens zeigen, daß das Glycogen der E. coli Zellen, die mit den Plasmiden pACAC + pRL2 transformiert worden waren, eine bis zu 290 ± 25% erhöhte Phosphorylierung an C-6-Position der Glucose aufweist, verglichen mit dem Kontrollansatz (E. coli Zellen transformiert mit dem Plasmid pACYC) (siehe folgende Tabelle)

| | |
|---|---|
| E. coli-Zellen | Glucose-6-Phosphat:Glucose im Glycogen |
| E. coli-K1 | 1:(4600 ± 1150) |
| E. coli-K2 | 1:(1570 ± 390) |

Die hier dargestellten Phosphorylierungsgrade sind der Mittelwert aus mindestens 6 Messungen ausgehend von 6 unabhängigen Transformationen und Glycogenisolierungen.

### Beispiel 10

### Einführung des Plasmids p35S-anti-RL in Kombination mit dem Plasmid p35SH-anti-BE in das Genom von Kartoffelpflanzen

Das Plasmid p35S-anti-RL wurde konstruiert wie im Beispiel 6 beschrieben. Das Plasmid p35SH-anti-BE wurde konstruiert wie in der Anmeldung WO95/07355, Beispiel 3, beschrieben. Beide Plasmide wurden mit Hilfe der Agrobakterium vermittelter Transformation wie oben beschrieben sequentiell in Kartoffelpflanzen transferiert. Dazu wurde zunächst das Plasmid p35SH-anti-BE in Kartoffelpflanzen transformiert. Es wurden ganze Pflanzen regeneriert und auf eine verringerte Expression des branching-Enzymgens selektiert. Anschließend wurde das Plasmid p35S-anti-RL in die schon reduzierte Expression des *branching*-Enzyms aufweisenden transgenen Pflanzen transformiert. Aus den transformierten Zellen wurden wiederum transgene Pflanzen regeneriert, und die transformierten Pflanzen wurden unter Gewächshausbedingungen kultiviert. Die Überprüfung des Erfolges der genetischen Veränderung der Pflanzen in Bezug auf eine stark reduzierte Expression sowohl des *branching*-Enzymgens als auch in Bezug auf eine stark reduzierte Expression des RL-Gens erfolgte durch Analyse der gesamten RNA in einer RNA-Blot-Analyse bezüglich des Verschwindens der zu Verzweigungsenzym-cDNA bzw. RL-cDNA komplementären Transkripte. Hierzu wurde die Gesamt-RNA aus Blätter transformierten Pflanzen nach beschriebenen Methoden isoliert, gelelektrophoretisch aufgetrennt, auf eine Membran transferiert und mit einer radioaktiv markierten Probe hybridisiert, die die unter Seq ID No. 1 dargestellte Sequenz oder einen Teil dieser Sequenz aufweist und anschließend mit einer radioaktiv markierten Probe hybridisiert, die die Sequenz der Verzweigungsenzym-cDNA (vgl. WO92/14827, Beispiel 1) oder einen Teil derselben aufweist. In ca. 5% - 10% der transformierten Pflanzen fehlte in der RNA-Blot-Analyse sowohl die Bande, die das spezifische Transkript der unter Seq. ID No. 1 dargestellten Sequenz darstellt als auch die Bande, die das spezifische Transkript der Verzweigungsenzym-cDNA (vgl. WO92/14827, Beispiel 1) darstellt. Diese Pflanzen, welche als R4-Pflanzen bezeichnet wurden, wurden zur Analyse der Qualität der in den Knollen enthaltenen Stärke eingesetzt.

### Beispiel 11

### Einführung des Plasmids pB33-anti-RL in Kombination mit dem Plasmid pB33-anti-GBSSI in das Genom von Kartoffelpflanzen

Das Plasmid pB33-anti-RL wurde konstruiert wie im Beispiel 7 beschrieben. Das Plasmid pB33-anti-GBSSI wurde wie folgt konstruiert:
Das *Dra*I/*Dra*I Fragment aus der Promotorregion des Patatin Klasse I Gens B33 von *Solanum* tuberosum, umfassend die Nukleotide -1512 bis +14 (Rocha-Sosa et al., EMBO J 8 (1989), 23-29) wurde in die SmaI Schnittstelle des Plasmids pUC19 ligiert. Aus dem entstandenen Plasmid wurde das Promotorfragment als *Eco*RI/*Hind*III Fragment in die *polylinker* Region des Plasmids pBin19 (Bevan, Nucleic Acids Research 12 (1984), 8711-8721) ligiert. Anschließend wurde das 3' EcoRI Fragment 1181 bis 2511 des GBSSI-Gens von *Solanum* tuberosum (Hergersberg, Dissertation (1988) Universität zu Köln) in die EcoRI Schnittstelle des entstandenen Plasmids ligiert.

Beide Plasmide wurden mit Hilfe Agrobakterium vermittelter Transformation sequentiell in Kartoffelpflanzen transferiert wie unter Beispiel 10 beschrieben. Aus den transformierten Zellen wurden ganze Pflanzen regeneriert, und die transformierten Pflanzen wurden unter Gewächshausbedingungen kultiviert. Die Überprüfung des Erfolges der genetischen Veränderungen der Pflanzen erfolgte durch Analyse der Gesamt-RNA in einer RNA-Blot-Analyse bezüglich des Verschwindens der zu den beiden cDNAs komplementären Transkripte. Hierzu wurde die Gesamt-RNA aus Knollen transformierter Pflanzen nach Standardmethoden isoliert, gelelektrophoretisch auf einem Agarosegel aufgetrennt, auf eine Membran transferiert und mit einer radioaktiv markierten Probe hybridisiert, die die unter Seq ID No. 1 dargestellte Sequenz oder einen Teil der Sequenz aufweist. Danach wurde die gleiche Membran mit einer radioaktiv markierten Probe hybridisiert, die die Sequenz des GBSSI-Gens oder einen Teil dieser Sequenz aufweist (Hergersberg, Dissertation (1988) Universität zu Köln). In ca. 5% bis 10% der transformierten Pflanzen fehlte in der RNA-Blot-Analyse die Bande, die Transkripte darstellt, die mit der erfindungsgemäßen cDNA bzw. mit der GBSSI-cDNA hybridisierten. Aus den Knollen dieser Pflanzen, welche als R3-Pflanzen bezeichnet wurden, wurde Stärke isoliert und analysiert.

### Beispiel 12

### Stärkeanalyse der R4-Pflanzen

Die gemäß Beispiel 10 transformierten Kartoffelpflanzen wurden hinsichtlich der Eigenschaften der synthetisierten Stärke untersucht. Die Analysen wurden an verschiedenen Linien von Kartoffelpflanzen durchgeführt, die mit den beiden Plasmiden p35S-anti-RL und p35SH-anti-BE transformiert worden waren und die in der RNA-Blot-Analyse die Banden nicht mehr oder stark reduziert aufwiesen, die Transkripte darstellen, die mit den erfindungsgemäßen DNA-Sequenzen bzw. mit der Sequenz der Verzweigungs-cDNA hybridisieren.

### a) Bestimmung der Viskosität wäßriger Lösungen der Stärke

Zur Bestimmung der Viskosität der wäßrigen Lösungen der in transformierten Kartoffelpflanzen synthetisierten Stärke wurde aus Knollen von Pflanzen, die mit dem Plasmid p35S-anti-RL und mit dem Plasmid p35SH-anti-BE transformiert worden waren, Stärke nach Standardverfahren isoliert. Es wurden jeweils 2 g Stärke in 25 ml H₂O aufgenommen und für die Analyse in einem Rapid Visco Analyser (Newport Scientific Pty Ltd, Investment Support Group, Warriewood NSW 2102, Australien) verwendet. Der Betrieb des Gerätes erfolgte nach den Angaben des Herstellers. Zur Bestimmung der Viskosität der wäßrigen Lösung der Stärke wurde die Stärkesuspension zunächst von 50°C auf 95°C erhitzt mit einer Geschwindigkeit von 12°C pro min. Anschließend wurde die Temperatur für 2,5 min bei 95°C gehalten. Danach wurde die Lösung von 95°C auf 50°C abgekühlt mit einer Geschwindigkeit von 12°C pro min. Während der gesamten Dauer wurde die Viskosität bestimmt. Repräsentative Ergebnisse derartiger Messungen sind in Form von Kurven, in denen die Viskosität in Abhängigkeit von der Zeit dargestellt ist, wiedergegeben. Fig. 6 zeigt unter 1 eine typische RVA-Kurve für Stärke, die aus Wildtyp-Pflanzen der Kartoffelvarietät Désirée isoliert wurde. Linie 2 bzw. 3 zeigen typische RVA-Kurven für Stärken, die aus Kartoffelpflanzen isoliert wurde, die mit dem Plasmid p35SH-anti-BE bzw. p35S-anti-RL transformiert worden waren. Linie 4 zeigt eine typische RVA-Kurve für Stärke, die aus den Knollen von Pflanzen isoliert worden ist, die mit dem Plasmid p35SH- anti-BE in Kombination mit dem Plasmid p35S-anti-RL transformiert worden ist. Linie 4 zeichnet sich durch das Fehlen jedweder Viskositätszunahme in Abhängigkeit von der Temperatur aus.

### b) Bestimmung des Amylose/Amylopektinverhältnisses

Aus den Knollen von transformierten Kartoffelpflanzen isolierte Stärke wurde auf das Amylose zu Amylopektinverhältnis untersucht. Dabei ergab sich für die Pflanzenlinie R4-1 (dargestellt in Linie 4 der Fig. 6) ein Amylosegehalt von über 70%. Für die Pflanzenlinie R4-3 ergab sich ein Amylosewert von 27%, während der Amylosegehalt in Wildtypstärke aus der Sorte Désirée zwischen 19 und 22% liegt.

### Beispiel 13

### Stärkeanalyse der R3-Pflanzen

Die gemäß Beispiel 11 transformierten Kartoffelpflanzen wurden hinsichtlich der Eigenschaften der synthetisierten Stärke untersucht. Die Analysen wurden an verschiedenen Linien von Kartoffelpflanzen durchgeführt, die mit den beiden Plasmiden pB33-anti-RL und pB33-anti-GBSSI transformiert worden waren und die in der RNA-Blot-Analyse die Banden nicht mehr oder stark reduziert aufwiesen, die Transkripte darstellen, die mit den erfindungsgemäßen DNA-Sequenzen bzw. mit der Sequenz der GBSSI-cDNA hybridisieren.
a) Bestimmung der Viskosität wäßriger Lösungen der Stärke
Zur Bestimmung der Viskosität der wäßrigen Lösungen der in transformierten Kartoffelpflanzen synthetisierten Stärke wurde aus Knollen von Pflanzen, die mit dem Plasmid pB33-anti-RL in Kombination mit dem Plasmid pB33-anti-GBSSI transformiert worden waren, Stärke nach Standardverfahren isoliert. Die Bestimmung der Viskosität mittels eines Rapid Visco Analysers erfolgte nach der in Beispiel 12, Teil a, beschriebenen Methode. Die Ergebnisse sind in Figur 7 dargestellt. Fig. 7 zeigt in Linie 1 eine typische RVA-Kurve für Stärke, die aus Wildtyp-Pflanzen der Kartoffelvarietät Désirée isoliert wurde. Linie 2 bzw. 3 zeigen typische RVA-Kurven für Stärken, die aus Kartoffelpflanzen isoliert wurde, die mit dem Plasmid pB33-anti-GBSSI bzw. p35S-anti-RL transformiert worden waren. Linie 4 zeigt eine typische RVA-Kurve für Stärke, die aus den Kartoffelpflanzen isoliert wurde, die mit dem Plasmid pB33-anti-GBSSI in Kombination mit dem Plasmid pB33-anti-RL transformiert worden waren. Diese Kurve zeichnet sich durch das Fehlen des Viskositätsmaximums sowie dem Fehlen des Anstiegs der Viskosität bei 50°C aus. Des weiteren zeichnet sich diese Stärke dadurch aus, daß der nach RVA-Behandlung erhaltene Kleister so gut wie keine Retrogradation nach mehrtägiger Inkubation bei Raumtemperatur aufweist.
b) Bestimmung des Amylose/Amylopektinverhältnisses
Aus den Knollen von transformierten Kartoffelpflanzen isolierte Stärke wurde auf das Amylose zu Amylopektinverhältnis untersucht. Dabei ergab sich für die Pflanzenlinie R3-5 (dargestellt in Linie 4 der Fig. 7) ein Amylosegehalt von unter 4%, für die Pflanzenlinie R3-6 ein Amylosegehalt von unter 3%. Der Amylosegehalt in Wildtypstärke aus der Sorte Désirée liegt zwischen 19 und 22% liegt.
c) Bestimmung des Phosphatgehaltes der Stärke
Der Phosphatgehalt der Stärke wurde bestimmt, indem die Menge an Phosphat, das an der C-6-Position von Glucoseresten gebunden war, gemessen wurde. Hierzu wurde Stärke zunächst durch Säurehydrolyse gespalten und anschließend der Gehalt an Glucose-6-Phosphat mittels eines Enzymtests bestimmt, wie im folgenden beschrieben.
100 mg Stärke wurden in 500 µl 0,7 N HCl 4 h bei 100 °C inkubiert. Nach der Säurehydrolyse wurden 10 µl des Ansatzes in 600 µl Imidazolpuffer (100 mM Imidazol, 5 mM MgCl₂, pH 6,9, 0,4 mM NAD⁺) gegeben. Die Bestimmung der Menge an Glucose-6-Phosphat in dem Ansatz erfolgte durch Umsetzung mit dem Enzym Glucose-6-Phosphat-Dehydrogenase. Dazu wurde dem Ansatz 1 U Glucose-6-Phosphat-Dehydrogenase (aus Leuconostoc mesenteroides (Boehringer Mannheim)) zugesetzt und die Menge an gebildetem NADH durch Messung der Absorption bei 340 nm bestimmt.
Der Gehalt an Glucose-6-Phosphat/Milligramm Stärke ist in der folgenden Tabelle für nicht-transformierte Kartoffelpflanzen der Varietät Désirée sowie für die Linien R3-5 und R3-6 transgener Kartoffelpflanzen, die mit dem Plasmid pB33-anti-RL in Kombination mit dem Plasmid pB33-anti-GBSSI transformiert worden waren, angegeben. Zum Vergleich ist der Wert für die Stärke aus der sog. *waxy*-Kartoffel (US2-10) mit angegeben, die mit dem Plasmid pB33-anti-GBSSI transformiert worden war.

**Tabelle 7**

| Pflanzen | nmol Glucose-6-Phosphat/mg Stärke | % |
|---|---|---|
| Wildtyp | 9,80 ± 0,68 | 100 |
| R3-5 | 1,32 ± 0,10 | 13 |
| R3-6 | 1,37 ± 0,15 | 14 |
| US2-10 | 10,82 ± 0,42 | 110 |

## Patentansprüche

1. Kartoffel-Stärke, die einen nach der Methode von Hovenkamp-Hermelink et al. (Potato Research 31, (1988) 241-246) bestimmten Amylosegehalt von über 27% aufweist.

2. Kartoffel-Stärke nach Anspruch 1, wobei der nach der Methode von Hovenkamp-Hermelink et al. (Potato Research 31, (1988) 241-246) bestimmte Amylosegehalt über 50% ist.

3. Kartoffel-Stärke nach Anspruch 2, wobei der nach der Methode von Hovenkamp-Hermelink et al. (Potato Research 31, (1988) 241-246) bestimmte Amylosegehalt über 60% ist

4. Nucleinsäuremolekül, das für ein Protein codiert, das in pflanzlichen Zellen sowohl an Stärkekörner gebunden vorliegt, als auch in löslicher Form, ausgewählt aus der Gruppe bestehend aus:
(a) Nucleinsäuremolekülen, die für ein Protein mit der unter SEQ ID No. 2 angegebenen Aminosäuresequenz codieren;
(b) Nucleinsäuremolekülen, die die codierende Region der unter SEQ ID No. 1 angegebenen Nucleotidsequenz umfassen;
(c) Nucleinsäuremolekülen, die mit den unter (a) oder (b) genannten Nucleinsäuremolekülen hybridisieren;
(d) Nucleinsäuremolekülen, deren Sequenz aufgrund des genetischen Codes degeneriert ist im Vergleich zu den Sequenzen der unter (a), (b) oder (c) genannten Nucleinsäuremoleküle; und
(e) Fragmenten, Derivaten oder allelischen Varianten der unter (a) bis (d) genannten Nucleinsäuremoleküle.

5. Vektor enthaltend ein Nucleinsäuremolekül nach Anspruch 4.

6. Vektor nach Anspruch 5, wobei das Nucleinsäuremolekül verknüpft ist mit regulatorischen Elementen, die die Transkription in eukaryontischen oder prokaryontischen Zellen gewährleisten.

7. Wirtszelle, die genetisch modifiziert ist mit einem Nucleinsäuremolekül nach Anspruch 4 oder einem Vektor nach Anspruch 5 oder 6.

8. Wirtszelle nach Anspruch 7, die eine transgene Pflanzenzelle ist.

9. Pflanze enthaltend Pflanzenzellen nach Anspruch 8.

10. Stärke erhältlich aus Pflanzenzellen nach Anspruch 8 oder einer Pflanze nach Anspruch 9.

11. Verfahren zur Herstellung eines Proteins, das in pflanzlichen Zellen sowohl an Stärkekörner gebunden vorliegt als auch in löslicher Form, bei dem eine Wirtszelle nach Anspruch 7 unter Bedingungen kultiviert wird, die die Expression des Proteins erlauben, und das Protein aus den Zellen und/oder dem Kulturmedium isoliert wird.

12. Protein, das durch ein Nucleinsäuremolekül nach Anspruch 4 codiert wird oder das erhältlich ist durch ein Verfahren nach Anspruch 11.

13. Antikörper, der spezifisch ein Protein nach Anspruch 12 erkennt.

14. Nucleinsäuremolekül von mindestens 15 Nucleotiden Länge, das spezifisch mit einem Nucleinsäuremolekül nach Anspruch 4 hybridisiert.

15. DNA-Molekül codierend eine antisense-RNA, die komplementär ist zu transkripten eines DNA-Moleküls nach Anspruch 4.

16. DNA-Molekül codierend eine RNA mit Ribozymaktivität, die spezifisch Transkripte eines DNA-Moleküls nach Anspruch 4 spaltet.

17. DNA-Molekül, codierend eine RNA, die bei Expression in einer pflanzlichen Zelle aufgrund eines Cosuppressions-Effektes zur Verringerung der Expression eines Nucleinsäuremoleküls nach Anspruch 4 führt.

18. Vektor enthaltend ein DNA-Molekül nach einem der Ansprüche 15 bis 17.

19. Vektor nach Anspruch 18, wobei das DNA-Molekül kombiniert ist mit regulatorischen DNA-Elementen, die die Transkription in pflanzlichen Zellen gewährleisten.

20. Wirtszelle enthaltend ein DNA-Molekül nach einem der Ansprüche 15 bis 17 oder einen Vektor nach Anspruch 18 oder 19.

21. Transgene Pflanzenzelle enthaltend ein DNA-Molekül nach einem der Ansprüche 15 bis 17 in Kombination mit regulatorischen DNA-Elementen, die die Transkription in pflanzlichen Zellen gewährleisten.

22. Transgene Pflanzenzelle nach Anspruch 21, bei der die Aktivität mindestens eines weiteren, an der Stärkebiosynthese oder -modifikation beteiligten Enzyms verringert ist im Vergleich zu nichtransformierten Pflanzen.

23. Transgene Pflanzenzelle nach Anspruch 22, bei der die Aktivität eines Verzweigungsenzyms verringert ist.

24. Transgene Pflanzenzelle nach Anspruch 23, bei der die Aktivität einer stärkekorngebundenen Stärkesynthase der Isoform I (GBSSI) verringert ist.

25. Transgene Pflanze erhältlich durch Regeneration einer Pflanzenzelle nach einem der Ansprüche 21 bis 24.

26. Stärke erhältlich aus Pflanzenzellen nach einem der Ansprüche 21 bis 24 oder Pflanzen nach Anspruch 25.

27. RNA-Molekül erhältlich durch Transkription eines DNA Moleküls nach einem der Ansprüche 15 bis 17.

28. Verfahren zur Herstellung von transgenen Pflanzenzellen, die eine modifizierte Stärke synthetisieren, **dadurch gekennzeichnet, dass** in den Zellen die Menge von Proteinen nach Anspruch 12 verringert wird, die endogen in der Zelle synthetisiert werden.

29. Verfahren nach Anspruch 28, **dadurch gekennzeichnet, dass** die Verringerung der Menge der Proteine nach Anspruch 12 in den Zellen durch einen antisense-Effekt erzielt wird.

30. Verfahren nach Anspruch 28, **dadurch gekennzeichnet, dass** die Verringerung der Menge der Proteine nach Anspruch 12 in den Zellen durch einen Ribozymeffekt erzielt wird.

31. Verfahren nach Anspruch 28, **dadurch gekennzeichnet, dass** die Verringerung der Menge der Proteine nach Anspruch 12 in den Zellen durch einen Cosuppressions-Effekt erzielt wird.

32. Verfahren nach einem der Ansprüche 28 bis 31, wobei die Enzymaktivität mindestens eines weiteren an der Stärkebiosynthese und/oder Modifikation beteiligten Enzyms reduziert wird.

33. Verfahren nach Anspruch 32, wobei das Enzym ein Verzweigungsenzym ist.

34. Verfahren nach Anspruch 32, wobei das Enzym eine stärkekorngebundene Stärkesynthase der Isoform I (GBSSI) ist.

35. Pflanzenzelle erhältlich durch ein Verfahren nach einem der Ansprüche 28 bis 34.

36. Transgene Pflanze erhältlich durch Regeneration der Pflanzenzelle nach Anspruch 35.

37. Stärke erhältlich aus Pflanzenzellen nach Anspruch 35 oder einer Pflanze nach Anspruch 36.

38. Stärke nach Anspruch 37, **dadurch gekennzeichnet, dass** sie aus Kartoffel stammt.

39. Vermehrungsmaterial von Pflanzen nach Anspruch 9 enthaltend Pflanzenzellen nach Anspruch 8.

40. Vermehrungsmaterial von Pflanzen nach Anspruch 25 oder 36, enthaltend Pflanzenzellen nach einem der Ansprüche 21 bis 24 oder nach Anspruch 35.

41. Transgene Pflanze nach Anspruch 25 oder 36, die eine Kartoffelpflanze ist.

42. Knolle einer Kartoffelpflanze nach Anspruch 41.

43. Knolle nach Anspruch 42, die im Vergleich zu Knollen von Wildtyp-Pflanzen ein verringertes "cold sweetening" aufweist.


SEQUENZPROTOKOLL

(1) ALLGEMEINE ANGABEN:

(i) ANMELDER:
(A) NAME: Jens Koßmann
(B) STRASSE: Golmer Fichten 9
(C) ORT: Golm
(E) LAND: DE
(F) POSTLEITZAHL: 14476

(ii) BEZEICHNUNG DER ERFINDUNG: Pflanzen, die eine modifizierte Staerke
synthetisieren, Verfahren zu ihrer Herstellung, sowie
modifizierte Stärke

(iii) ANZAHL DER SEQUENZEN: 4

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 4856 Basenpaare
(B) ART: Nucleotid
(C) STRANGFORM: Einzelstrang
(D) TOPOLOGIE: linear

(ii) ART DES MOLEKÜLS: cDNA zu mRNA

(vi) URSPRÜNLICHE HERKUNFT:
(A) ORGANISMUS: Solanum tuberosum
(B) STAMM: C.V. Berolina

(ix) MERKMAL:
(A) NAME/SCHLÜSSEL: CDS
(B) LAGE:105..4497

(xi) SEQUENZBESCHREIBUNG: SEQ ID NO: 1:

CATCTTCATC GAATTTCTCG AAGCTTCTTC GCTAATTTCC TGGTTTCTTC ACTCAAAATC 60

GACGTTTCTA GCTGAACTTG AGTGAATTAA GCCAGTGGGA GGAT ATG AGT AAT TCC 116
Met Ser Asn Ser
1

TTA GGG AAT AAC TTG CTG TAC CAG GGA TTC CTA ACC TCA ACA GTG TTG 164
Leu Gly Asn Asn Leu Leu Tyr Gln Gly Phe Leu Thr Ser Thr Val Leu
5                   10                  15                  20

GAA CAT AAA AGT AGA ATC AGT CCT CCT TGT GTT GGA GGC AAT TCT TTG 212
Glu His Lys Ser Arg Ile Ser Pro Pro Cys Val Gly Gly Asn Ser Leu
                25                  30                  35

TTT CAA CAA CAA GTG ATC TCG AAA TCA CCT TTA TCA ACT GAG TTT CGA 260
Phe Gln Gln Gln Val Ile Ser Lys Ser Pro Leu Ser Thr Glu Phe Arg
            40                  45                  50

GGT AAC AGG TTA AAG GTG CAG AAA AAG AAA ATA CCT ATG GAA AAG AAG 308
Gly Asn Arg Leu Lys Val Gln Lys Lys Lys Ile Pro Met Glu Lys Lys
        55                  60                  65

CGT GCT TTT TCT AGT TCT CCT CAT GCT GTA CTT ACC ACT GAT ACC TCT 356
Arg Ala Phe Ser Ser Ser Pro His Ala Val Leu Thr Thr Asp Thr Ser
    70                  75                  80

TCT GAG CTA GCA GAA AAG TTC AGT CTA GGG GGG AAT ATT GAG CTA CAG 404
Ser Glu Leu Ala Glu Lys Phe Ser Leu Gly Gly Asn Ile Glu Leu Gln
85                  90                  95                  100

GTT GAT GTT AGG CCT CCC ACT TCA GGT GAT GTG TCC TTT GTG GAT TTT 452
Val Asp Val Arg Pro Pro Thr Ser Gly Asp Val Ser Phe Val Asp Phe
                105                 110                 115

CAA GTA ACA AAT GGT AGT GAT AAA CTG TTT TTG CAC TGG GGG GCA GTA 500
Gln Val Thr Asn Gly Ser Asp Lys Leu Phe Leu His Trp Gly Ala Val
            120                 125                 130

AAA TTC GGG AAA GAA ACA TGG TCT CTT CCG AAT GAT CGT CCA GAT GGG 548
Lys Phe Gly Lys Glu Thr Trp Ser Leu Pro Asn Asp Arg Pro Asp Gly
        135                 140                 145

ACC AAA GTG TAC AAG AAC AAA GCA CTT AGA ACT CCA TTT GTT AAA TCT 596
Thr Lys Val Tyr Lys Asn Lys Ala Leu Arg Thr Pro Phe Val Lys Ser
    150                 155                 160

GGC TCT AAC TCC ATC CTG AGA CTG GAG ATA CGA GAC ACT GCT ATC GAA 644
Gly Ser Asn Ser Ile Leu Arg Leu Glu Ile Arg Asp Thr Ala Ile Glu
165                 170                 175                 180

GCT ATT GAG TTT CTC ATA TAC GAT GAA GCC CAC GAT AAA TGG ATA AAG 692
Ala Ile Glu Phe Leu Ile Tyr Asp Glu Ala His Asp Lys Trp Ile Lys
                185                 190                 195

AAT AAT GGT GGT AAT TTT CGT GTC AAA TTG TCA AGA AAA GAG ATA CGA 740
Asn Asn Gly Gly Asn Phe Arg Val Lys Leu Ser Arg Lys Glu Ile Arg
            200                 205                 210

GGC CCA GAT GTT TCT GTT CCT GAG GAG CTT GTA CAG ATC CAA TCA TAT 788
Gly Pro Asp Val Ser Val Pro Glu Glu Leu Val Gln Ile Gln Ser Tyr
        215                 220                 225

TTG AGG TGG GAG AGG AAG GGA AAA CAG AAT TAC CCC CCT GAG AAA GAG 836
Leu Arg Trp Glu Arg Lys Gly Lys Gln Asn Tyr Pro Pro Glu Lys Glu
    230                 235                 240

AAG GAG GAA TAT GAG GCT GCT CGA ACT GTG CTA CAG GAG GAA ATA GCT 884
Lys Glu Glu Tyr Glu Ala Ala Arg Thr Val Leu Gln Glu Glu Ile Ala
245                 250                 255                 260

CGT GGT GCT TCC ATA CAG GAC ATT CGA GCA AGG CTA ACA AAA ACT AAT 932
Arg Gly Ala Ser Ile Gln Asp Ile Arg Ala Arg Leu Thr Lys Thr Asn
                265                 270                 275

GAT AAA AGT CAA AGC AAA GAA GAG CCT CTT CAT GTA ACA AAG AGT GAT 980
Asp Lys Ser Gln Ser Lys Glu Glu Pro Leu His Val Thr Lys Ser Asp
            280                 285                 290

ATA CCT GAT GAC CTT GCC CAA GCA CAA GCT TAC ATT AGG TGG GAG AAA 1028
Ile Pro Asp Asp Leu Ala Gln Ala Gln Ala Tyr Ile Arg Trp Glu Lys
        295                 300                 305

GCA GGA AAG CCG AAC TAT CCT CCA GAA AAG CAA ATT GAA GAA CTC GAA 1076
Ala Gly Lys Pro Asn Tyr Pro Pro Glu Lys Gln Ile Glu Glu Leu Glu
    310                 315                 320

GAA GCA AGA AGA GAA TTG CAA CTT GAG CTT GAG AAA GGC ATT ACC CTT 1124
Glu Ala Arg Arg Glu Leu Gln Leu Glu Leu Glu Lys Gly Ile Thr Leu
325                 330                 335                 340

GAT GAG TTG CGG AAA ACG ATT ACA AAA GGG GAG ATA AAA ACT AAG GTG 1172
Asp Glu Leu Arg Lys Thr Ile Thr Lys Gly Glu Ile Lys Thr Lys Val
                345                 350                 355

GAA AAG CAC CTG AAA AGA AGT TCT TTT GCC GTT GAA AGA ATC CAA AGA 1220
Glu Lys His Leu Lys Arg Ser Ser Phe Ala Val Glu Arg Ile Gln Arg
            360                 365                 370

AAG AAG AGA GAC TTT GGG CAT CTT ATT AAT AAG TAT ACT TCC AGT CCT 1268
Lys Lys Arg Asp Phe Gly His Leu Ile Asn Lys Tyr Thr Ser Ser Pro
        375                 380                 385

GCA GTA CAA GTA CAA AAG GTC TTG GAA GAA CCA CCA GCC TTA TCT AAA 1316
Ala Val Gln Val Gln Lys Val Leu Glu Glu Pro Pro Ala Leu Ser Lys
    390                 395                 400

ATT AAG CTG TAT GCC AAG GAG AAG GAG GAG CAG ATT GAT GAT CCG ATC 1364
Ile Lys Leu Tyr Ala Lys Glu Lys Glu Glu Gln Ile Asp Asp Pro Ile
405                 410                 415                 420

CTA AAT AAA AAG ATC TTT AAG GTC GAT GAT GGG GAG CTA CTG GTA CTG 1412
Leu Asn Lys Lys Ile Phe Lys Val Asp Asp Gly Glu Leu Leu Val Leu
                425                 430                 435

GTA GCA AAG TCC TCT GGG AAG ACA AAA GTA CAT CTA GCT ACA GAT CTG 1460
Val Ala Lys Ser Ser Gly Lys Thr Lys Val His Leu Ala Thr Asp Leu
            440                 445                 450

AAT CAG CCA ATT ACT CTT CAC TGG GCA TTA TCC AAA AGT CCT GGA GAG 1508
Asn Gln Pro Ile Thr Leu His Trp Ala Leu Ser Lys Ser Pro Gly Glu
        455                 460                 465

TGG ATG GTA CCA CCT TCA AGC ATA TTG CCT CCT GGG TCA ATT ATT TTA 1556
Trp Met Val Pro Pro Ser Ser Ile Leu Pro Pro Gly Ser Ile Ile Leu
    470                 475                 480

GAC AAG GCT GCC GAA ACA CCT TTT TCA GCC AGT TCT TCT GAT GGT CTA 1604
Asp Lys Ala Ala Glu Thr Pro Phe Ser Ala Ser Ser Ser Asp Gly Leu
485                 490                 495                 500

ACT TCT AAG GTA CAA TCT TTG GAT ATA GTA ATT GAA GAT GGC AAT TTT 1652
Thr Ser Lys Val Gln Ser Leu Asp Ile Val Ile Glu Asp Gly Asn Phe
                505                 510                 515

GTG GGG ATG CCA TTT GTT CTT TTG TCT GGT GAA AAA TGG ATT AAG AAC 1700
Val Gly Met Pro Phe Val Leu Leu Ser Gly Glu Lys Trp Ile Lys Asn
            520                 525                 530

CAA GGG TCG GAT TTC TAT GTT GGC TTC AGT GCT GCA TCC AAA TTA GCA 1748
Gln Gly Ser Asp Phe Tyr Val Gly Phe Ser Ala Ala Ser Lys Leu Ala
        535                 540                 545

CTC AAG GCT GCT GGG GAT GGC AGT GGA ACT GCA AAG TCT TTA CTG GAT 1796
Leu Lys Ala Ala Gly Asp Gly Ser Gly Thr Ala Lys Ser Leu Leu Asp
    550                 555                 560

AAA ATA GCA GAT ATG GAA AGT GAG GCT CAG AAG TCA TTT ATG CAC CGG 1844
Lys Ile Ala Asp Met Glu Ser Glu Ala Gln Lys Ser Phe Met His Arg
565                 570                 575                 580

TTT AAT ATT GCA GCT GAC TTG ATA GAA GAT GCC ACT AGT GCT GGT GAA 1892
Phe Asn Ile Ala Ala Asp Leu Ile Glu Asp Ala Thr Ser Ala Gly Glu
                585                 590                 595

CTT GGT TTT GCT GGA ATT CTT GTA TGG ATG AGG TTC ATG GCT ACA AGG 1940
Leu Gly Phe Ala Gly Ile Leu Val Trp Met Arg Phe Met Ala Thr Arg
            600                 605                 610

CAA CTG ATA TGG AAC AAA AAC TAT AAC GTA AAA CCA CGT GAA ATA AGC 1988
Gln Leu Ile Trp Asn Lys Asn Tyr Asn Val Lys Pro Arg Glu Ile Ser
        615                 620                 625

AAG GCT CAG GAC AGA CTT ACA GAC TTG TTG CAG AAT GCT TTC ACC AGT 2036
Lys Ala Gln Asp Arg Leu Thr Asp Leu Leu Gln Asn Ala Phe Thr Ser
    630                 635                 640

CAC CCT CAG TAC CGT GAA ATT TTG CGG ATG ATT ATG TCA ACT GTT GGA 2084
His Pro Gln Tyr Arg Glu Ile Leu Arg Met Ile Met Ser Thr Val Gly
645                 650                 655                 660

CGT GGA GGT GAA GGG GAT GTA GGA CAG CGA ATT AGG GAT GAA ATT TTG 2132
Arg Gly Gly Glu Gly Asp Val Gly Gln Arg Ile Arg Asp Glu Ile Leu
                665                 670                 675

GTC ATC CAG AGG AAC AAT GAC TGC AAG GGT GGT ATG ATG CAA GAA TGG 2180
Val Ile Gln Arg Asn Asn Asp Cys Lys Gly Gly Met Met Gln Glu Trp
            680                 685                 690

CAT CAG AAA TTG CAT AAT AAT ACT AGT CCT GAT GAT GTT GTG ATC TGT 2228
His Gln Lys Leu His Asn Asn Thr Ser Pro Asp Asp Val Val Ile Cys
        695                 700                 705

CAG GCA TTA ATT GAC TAC ATC AAG AGT GAT TTT GAT CTT GGT GTT TAT 2276
Gln Ala Leu Ile Asp Tyr Ile Lys Ser Asp Phe Asp Leu Gly Val Tyr
    710                 715                 720

TGG AAA ACC CTG AAT GAG AAC GGA ATA ACA AAA GAG CGT CTT TTG AGT 2324
Trp Lys Thr Leu Asn Glu Asn Gly Ile Thr Lys Glu Arg Leu Leu Ser
725                 730                 735                 740

TAT GAC CGT GCT ATC CAT TCT GAA CCA AAT TTT AGA GGA GAT CAA AAG 2372
Tyr Asp Arg Ala Ile His Ser Glu Pro Asn Phe Arg Gly Asp Gln Lys
                745                 750                 755

GGT GGT CTT TTG CGT GAT TTA GGT CAC TAT ATG AGA ACA TTG AAG GCA 2420
Gly Gly Leu Leu Arg Asp Leu Gly His Tyr Met Arg Thr Leu Lys Ala
            760                 765                 770

GTT CAT TCA GGT GCA GAT CTT GAG TCT GCT ATT GCA AAC TGC ATG GGC 2468
Val His Ser Gly Ala Asp Leu Glu Ser Ala Ile Ala Asn Cys Met Gly
        775                 780                 785

TAC AAA ACT GAG GGA GAA GGC TTT ATG GTT GGA GTC CAG ATA AAT CCT 2516
Tyr Lys Thr Glu Gly Glu Gly Phe Met Val Gly Val Gln Ile Asn Pro
    790                 795                 800

GTA TCA GGC TTG CCA TCT GGC TTT CAG GAC CTC CTC CAT TTT GTC TTA 2564
Val Ser Gly Leu Pro Ser Gly Phe Gln Asp Leu Leu His Phe Val Leu
805                 810                 815                 820

GAC CAT GTG GAA GAT AAA AAT GTG GAA ACT CTT CTT GAG AGA TTG CTA 2612
Asp His Val Glu Asp Lys Asn Val Glu Thr Leu Leu Glu Arg Leu Leu
                825                 830                 835

GAG GCT CGT GAG GAG CTT AGG CCC TTG CTT CTC AAA CCA AAC AAC CGT 2660
Glu Ala Arg Glu Glu Leu Arg Pro Leu Leu Leu Lys Pro Asn Asn Arg
            840                 845                 850

CTA AAG GAT CTG CTG TTT TTG GAC ATA GCA CTT GAT TCT ACA GTT AGA 2708
Leu Lys Asp Leu Leu Phe Leu Asp Ile Ala Leu Asp Ser Thr Val Arg
        855                 860                 865

ACA GCA GTA GAA AGG GGA TAT GAA GAA TTG AAC AAC GCT AAT CCT GAG 2756
Thr Ala Val Glu Arg Gly Tyr Glu Glu Leu Asn Asn Ala Asn Pro Glu
    870                 875                 880

AAA ATC ATG TAC TTC ATC TCC CTC GTT CTT GAA AAT CTC GCA CTC TCT 2804
Lys Ile Met Tyr Phe Ile Ser Leu Val Leu Glu Asn Leu Ala Leu Ser
885                 890                 895                 900

GTG GAC GAT AAT GAA GAT CTT GTT TAT TGC TTG AAG GGA TGG AAT CAA 2852
Val Asp Asp Asn Glu Asp Leu Val Tyr Cys Leu Lys Gly Trp Asn Gln
                905                 910                 915

GCT CTT TCA ATG TCC AAT GGT GGG GAC AAC CAT TGG GCT TTA TTT GCA 2900
Ala Leu Ser Met Ser Asn Gly Gly Asp Asn His Trp Ala Leu Phe Ala
            920                 925                 930

AAA GCT GTG CTT GAC AGA ACC CGT CTT GCA CTT GCA AGC AAG GCA GAG 2948
Lys Ala Val Leu Asp Arg Thr Arg Leu Ala Leu Ala Ser Lys Ala Glu
        935                 940                 945

TGG TAC CAT CAC TTA TTG CAG CCA TCT GCC GAA TAT CTA GGA TCA ATA 2996
Trp Tyr His His Leu Leu Gln Pro Ser Ala Glu Tyr Leu Gly Ser Ile
    950                 955                 960

CTT GGG GTG GAC CAA TGG GCT TTG AAC ATA TTT ACT GAA GAA ATT ATA 3044
Leu Gly Val Asp Gln Trp Ala Leu Asn Ile Phe Thr Glu Glu Ile Ile
965                 970                 975                 980

CGT GCT GGA TCA GCA GCT TCA TTA TCC TCT CTT CTT AAT AGA CTC GAT 3092
Arg Ala Gly Ser Ala Ala Ser Leu Ser Ser Leu Leu Asn Arg Leu Asp
                985                 990                 995

CCC GTG CTT CGG AAA ACT GCA AAT CTA GGA AGT TGG CAG ATT ATC AGT 3140
Pro Val Leu Arg Lys Thr Ala Asn Leu Gly Ser Trp Gln Ile Ile Ser
            1000                1005                1010

CCA GTT GAA GCC GTT GGA TAT GTT GTC GTT GTG GAT GAG TTG CTT TCA 3188
Pro Val Glu Ala Val Gly Tyr Val Val Val Val Asp Glu Leu Leu Ser
        1015                1020                1025

GTT CAG AAT GAA ATC TAC GAG AAG CCC ACG ATC TTA GTA GCA AAA TCT 3236
Val Gln Asn Glu Ile Tyr Glu Lys Pro Thr Ile Leu Val Ala Lys Ser
    1030                1035                1040

GTT AAA GGA GAG GAG GAA ATT CCT GAT GGT GCT GTT GCC CTG ATA ACA 3284
Val Lys Gly Glu Glu Glu Ile Pro Asp Gly Ala Val Ala Leu Ile Thr
1045                1050                1055                1060

CCA GAC ATG CCA GAT GTT CTT TCA CAT GTT TCT GTT CGA GCT AGA AAT 3332
Pro Asp Met Pro Asp Val Leu Ser His Val Ser Val Arg Ala Arg Asn
                1065                1070                1075

GGG AAG GTT TGC TTT GCT ACA TGC TTT GAT CCC AAT ATA TTG GCT GAC 3380
Gly Lys Val Cys Phe Ala Thr Cys Phe Asp Pro Asn Ile Leu Ala Asp
            1080                1085                1090

CTC CAA GCA AAG GAA GGA AGG ATT TTG CTC TTA AAG CCT ACA CCT TCA 3428
Leu Gln Ala Lys Glu Gly Arg Ile Leu Leu Leu Lys Pro Thr Pro Ser
        1095                1100                1105

GAC ATA ATC TAT AGT GAG GTG AAT GAG ATT GAG CTC CAA AGT TCA AGT 3476
Asp Ile Ile Tyr Ser Glu Val Asn Glu Ile Glu Leu Gln Ser Ser Ser
    1110                1115                1120

AAC TTG GTA GAA GCT GAA ACT TCA GCA ACA CTT AGA TTG GTG AAA AAG 3524
Asn Leu Val Glu Ala Glu Thr Ser Ala Thr Leu Arg Leu Val Lys Lys
1125                1130                1135                1140

CAA TTT GGT GGT TGT TAC GCA ATA TCA GCA GAT GAA TTC ACA AGT GAA 3572
Gln Phe Gly Gly Cys Tyr Ala Ile Ser Ala Asp Glu Phe Thr Ser Glu
                1145                1150                1155

ATG GTT GGA GCT AAA TCA CGT AAT ATT GCA TAT CTG AAA GGA AAA GTG 3620
Met Val Gly Ala Lys Ser Arg Asn Ile Ala Tyr Leu Lys Gly Lys Val
            1160                1165                1170

CCT TCC TCG GTG GGA ATT CCT ACG TCA GTA GCT CTT CCA TTT GGA GTC 3668
Pro Ser Ser Val Gly Ile Pro Thr Ser Val Ala Leu Pro Phe Gly Val
        1175                1180                1185

TTT GAG AAA GTA CTT TCA GAC GAC ATA AAT CAG GGA GTG GCA AAA GAG 3716
Phe Glu Lys Val Leu Ser Asp Asp Ile Asn Gln Gly Val Ala Lys Glu
    1190                1195                1200

TTG CAA ATT CTG ATG AAA AAA CTA TCT GAA GGA GAC TTC AGC GCT CTT 3764
Leu Gln Ile Leu Met Lys Lys Leu Ser Glu Gly Asp Phe Ser Ala Leu
1205                1210                1215                1220

GGT GAA ATT CGC ACA ACG GTT TTA GAT CTT TCA GCA CCA GCT CAA TTG 3812
Gly Glu Ile Arg Thr Thr Val Leu Asp Leu Ser Ala Pro Ala Gln Leu
                1225                1230                1235

GTC AAA GAG CTG AAG GAG AAG ATG CAG GGT TCT GGC ATG CCT TGG CCT 3860
Val Lys Glu Leu Lys Glu Lys Met Gln Gly Ser Gly Met Pro Trp Pro
            1240                1245                1250

GGT GAT GAA GGT CCA AAG CGG TGG GAA CAA GCA TGG ATG GCC ATA AAA 3908
Gly Asp Glu Gly Pro Lys Arg Trp Glu Gln Ala Trp Met Ala Ile Lys
        1255                1260                1265

AAG GTG TGG GCT TCA AAA TGG AAT GAG AGA GCA TAC TTC AGC ACA AGG 3956
Lys Val Trp Ala Ser Lys Trp Asn Glu Arg Ala Tyr Phe Ser Thr Arg
    1270                1275                1280

AAG GTG AAA CTG GAT CAT GAC TAT CTG TGC ATG GCT GTC CTT GTT CAA 4004
Lys Val Lys Leu Asp His Asp Tyr Leu Cys Met Ala Val Leu Val Gln
1285                1290                1295                1300

GAA ATA ATA AAT GCT GAT TAT GCA TTT GTC ATT CAC ACA ACC AAC CCA 4052
Glu Ile Ile Asn Ala Asp Tyr Ala Phe Val Ile His Thr Thr Asn Pro
                1305                1310                1315

TCT TCC GGA GAC GAC TCA GAA ATA TAT GCC GAG GTG GTC AGG GGC CTT 4100
Ser Ser Gly Asp Asp Ser Glu Ile Tyr Ala Glu Val Val Arg Gly Leu
            1320                1325                1330

GGG GAA ACA CTT GTT GGA GCT TAT CCA GGA CGT GCT TTG AGT TTT ATC 4148
Gly Glu Thr Leu Val Gly Ala Tyr Pro Gly Arg Ala Leu Ser Phe Ile
        1335                1340                1345

TGC AAG AAA AAG GAT CTC AAC TCT CCT CAA GTG TTA GGT TAC CCA AGC 4196
Cys Lys Lys Lys Asp Leu Asn Ser Pro Gln Val Leu Gly Tyr Pro Ser
    1350                1355                1360

AAA CCG ATC GGC CTT TTC ATA AAA AGA TCT ATC ATC TTC CGA TCT GAT 4244
Lys Pro Ile Gly Leu Phe Ile Lys Arg Ser Ile Ile Phe Arg Ser Asp
1365                1370                1375                1380

TCC AAT GGG GAA GAT TTG GAA GGT TAT GCC GGT GCT GGC CTC TAC GAC 4292
Ser Asn Gly Glu Asp Leu Glu Gly Tyr Ala Gly Ala Gly Leu Tyr Asp
                1385                1390                1395

AGT GTA CCA ATG GAT GAG GAG GAA AAA GTT GTA ATT GAT TAC TCT TCC 4340
Ser Val Pro Met Asp Glu Glu Glu Lys Val Val Ile Asp Tyr Ser Ser
            1400                1405                1410

GAC CCA TTG ATA ACT GAT GGT AAC TTC CGC CAG ACA ATC CTG TCC AAC 4388
Asp Pro Leu Ile Thr Asp Gly Asn Phe Arg Gln Thr Ile Leu Ser Asn
        1415                1420                1425

ATT GCT CGT GCT GGA CAT GCT ATC GAG GAG CTA TAT GGC TCT CCT CAA 4436
Ile Ala Arg Ala Gly His Ala Ile Glu Glu Leu Tyr Gly Ser Pro Gln
    1430                1435                1440

GAC ATT GAG GGT GTA GTG AGG GAT GGA AAG ATT TAT GTC GTT CAG ACA 4484
Asp Ile Glu Gly Val Val Arg Asp Gly Lys Ile Tyr Val Val Gln Thr
1445                1450                1455                1460

AGA CCA CAG ATG T GATTATATTC TCGTTGTATG TTGTTCAGAG AAGACCACAG 4537
Arg Pro Gln Met

ATGTGATCAT ATTCTCATTG TATCAGATCT GTGACCACTT ACCTGATACC TCCCATGAAG 4597

TTACCTGTAT GATTATACGT GATCCAAAGC CATCACATCA TGTTCACCTT CAGCTATTGG 4657

AGGAGAAGTG AGAAGTAGGA ATTGCAATAT GAGGAATAAT AAGAAAAACT TTGTAAAAGC 4717

TAAATTAGCT GGGTATGATA TAGGGAGAAA TGTGTAAACA TTGTACTATA TATAGTATAT 4777

ACACACGCAT TATGTATTGC ATTATGCACT GAATAATATC GCAGCATCAA AGAAGAAATC 4837

CTTTGGGTGG TTTCAAAAA 4856

(2) ANGABEN ZU SEQ ID NO: 2:

(i) SEQUENZKENNZEICHEN:
(A) LÄNGE: 1464 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 2:
   Met Ser Asn Ser Leu Gly Asn Asn Leu Leu Tyr Gln Gly Phe Leu Thr
   1 5 10 15
   Ser Thr Val Leu Glu His Lys Ser Arg Ile Ser Pro Pro Cys Val Gly
   20 25 30
   Gly Asn Ser Leu Phe Gln Gln Gln Val Ile Ser Lys Ser Pro Leu Ser
   35 40 45
   Thr Glu Phe Arg Gly Asn Arg Leu Lys Val Gln Lys Lys Lys Ile Pro
   50 55 60
   Met Glu Lys Lys Arg Ala Phe Ser Ser Ser Pro His Ala Val Leu Thr
   65 70 75 80
   Thr Asp Thr Ser Ser Glu Leu Ala Glu Lys Phe Ser Leu Gly Gly Asn
   85 90 95
   Ile Glu Leu Gln Val Asp Val Arg Pro Pro Thr Ser Gly Asp Val Ser
   100 105 110
   Phe Val Asp Phe Gln Val Thr Asn Gly Ser Asp Lys Leu Phe Leu His
   115 120 125
   Trp Gly Ala Val Lys Phe Gly Lys Glu Thr Trp Ser Leu Pro Asn Asp
   130 135 140
   Arg Pro Asp Gly Thr Lys Val Tyr Lys Asn Lys Ala Leu Arg Thr Pro
   145 150 155 160
   Phe Val Lys Ser Gly Ser Asn Ser Ile Leu Arg Leu Glu Ile Arg Asp
   165 170 175
   Thr Ala Ile Glu Ala Ile Glu Phe Leu Ile Tyr Asp Glu Ala His Asp
   180 185 190
   Lys Trp Ile Lys Asn Asn Gly Gly Asn Phe Arg Val Lys Leu Ser Arg
   195 200 205
   Lys Glu Ile Arg Gly Pro Asp Val Ser Val Pro Glu Glu Leu Val Gln
   210 215 220
   Ile Gln Ser Tyr Leu Arg Trp Glu Arg Lys Gly Lys Gln Asn Tyr Pro
   225 230 235 240
   Pro Glu Lys Glu Lys Glu Glu Tyr Glu Ala Ala Arg Thr Val Leu Gln
   245                 250                 255
   Glu Glu Ile Ala Arg Gly Ala Ser Ile Gln Asp Ile Arg Ala Arg Leu
               260                 265                 270
   Thr Lys Thr Asn Asp Lys Ser Gln Ser Lys Glu Glu Pro Leu His Val
           275                 280                 285
   Thr Lys Ser Asp Ile Pro Asp Asp Leu Ala Gln Ala Gln Ala Tyr Ile
       290                 295                 300
   Arg Trp Glu Lys Ala Gly Lys Pro Asn Tyr Pro Pro Glu Lys Gln Ile
   305                 310                 315                 320
   Glu Glu Leu Glu Glu Ala Arg Arg Glu Leu Gln Leu Glu Leu Glu Lys
                   325                 330                 335
   Gly Ile Thr Leu Asp Glu Leu Arg Lys Thr Ile Thr Lys Gly Glu Ile
               340                 345                 350
   Lys Thr Lys Val Glu Lys His Leu Lys Arg Ser Ser Phe Ala Val Glu
           355                 360                 365
   Arg Ile Gln Arg Lys Lys Arg Asp Phe Gly His Leu Ile Asn Lys Tyr
       370                 375                 380
   Thr Ser Ser Pro Ala Val Gln Val Gln Lys Val Leu Glu Glu Pro Pro
   385                 390                 395                 400
   Ala Leu Ser Lys Ile Lys Leu Tyr Ala Lys Glu Lys Glu Glu Gln Ile
                   405                 410                 415
   Asp Asp Pro Ile Leu Asn Lys Lys Ile Phe Lys Val Asp Asp Gly Glu
               420                 425                 430
   Leu Leu Val Leu Val Ala Lys Ser Ser Gly Lys Thr Lys Val His Leu
           435                 440                 445
   Ala Thr Asp Leu Asn Gln Pro Ile Thr Leu His Trp Ala Leu Ser Lys
       450                 455                 460
   Ser Pro Gly Glu Trp Met Val Pro Pro Ser Ser Ile Leu Pro Pro Gly
   465                 470                 475                 480
   Ser Ile Ile Leu Asp Lys Ala Ala Glu Thr Pro Phe Ser Ala Ser Ser
                   485                 490                 495
   Ser Asp Gly Leu Thr Ser Lys Val Gln Ser Leu Asp Ile Val Ile Glu
               500                 505                 510
   Asp Gly Asn Phe Val Gly Met Pro Phe Val Leu Leu Ser Gly Glu Lys
           515                 520                 525
   Trp Ile Lys Asn Gln Gly Ser Asp Phe Tyr Val Gly Phe Ser Ala Ala
       530                 535                 540
   Ser Lys Leu Ala Leu Lys Ala Ala Gly Asp Gly Ser Gly Thr Ala Lys
   545                 550                 555                 560
   Ser Leu Leu Asp Lys Ile Ala Asp Met Glu Ser Glu Ala Gln Lys Ser
                   565                 570                 575
   Phe Met His Arg Phe Asn Ile Ala Ala Asp Leu Ile Glu Asp Ala Thr
               580                 585                 590
   Ser Ala Gly Glu Leu Gly Phe Ala Gly Ile Leu Val Trp Met Arg Phe
           595                 600                 605
   Met Ala Thr Arg Gln Leu Ile Trp Asn Lys Asn Tyr Asn Val Lys Pro
       610                 615                 620
   Arg Glu Ile Ser Lys Ala Gln Asp Arg Leu Thr Asp Leu Leu Gln Asn
   625                 630                 635                 640
   Ala Phe Thr Ser His Pro Gln Tyr Arg Glu Ile Leu Arg Met Ile Met
                   645                 650                 655
   Ser Thr Val Gly Arg Gly Gly Glu Gly Asp Val Gly Gln Arg Ile Arg
               660                 665                 670
   Asp Glu Ile Leu Val Ile Gln Arg Asn Asn Asp Cys Lys Gly Gly Met
           675                 680                 685
   Met Gln Glu Trp His Gln Lys Leu His Asn Asn Thr Ser Pro Asp Asp
       690                 695                 700
   Val Val Ile Cys Gln Ala Leu Ile Asp Tyr Ile Lys Ser Asp Phe Asp
   705                 710                 715                 720
   Leu Gly Val Tyr Trp Lys Thr Leu Asn Glu Asn Gly Ile Thr Lys Glu
                   725                 730                 735
   Arg Leu Leu Ser Tyr Asp Arg Ala Ile His Ser Glu Pro Asn Phe Arg
               740                 745                 750
   Gly Asp Gln Lys Gly Gly Leu Leu Arg Asp Leu Gly His Tyr Met Arg
           755                 760                 765
   Thr Leu Lys Ala Val His Ser Gly Ala Asp Leu Glu Ser Ala Ile Ala
       770                 775                 780
   Asn Cys Met Gly Tyr Lys Thr Glu Gly Glu Gly Phe Met Val Gly Val
   785                 790                 795                 800
   Gln Ile Asn Pro Val Ser Gly Leu Pro Ser Gly Phe Gln Asp Leu Leu
                   805                 810                 815
   His Phe Val Leu Asp His Val Glu Asp Lys Asn Val Glu Thr Leu Leu
               820                 825                 830
   Glu Arg Leu Leu Glu Ala Arg Glu Glu Leu Arg Pro Leu Leu Leu Lys
           835                 840                 845
   Pro Asn Asn Arg Leu Lys Asp Leu Leu Phe Leu Asp Ile Ala Leu Asp
       850                 855                 860
   Ser Thr Val Arg Thr Ala Val Glu Arg Gly Tyr Glu Glu Leu Asn Asn
   865                 870                 875                 880
   Ala Asn Pro Glu Lys Ile Met Tyr Phe Ile Ser Leu Val Leu Glu Asn
                   885                 890                 895
   Leu Ala Leu Ser Val Asp Asp Asn Glu Asp Leu Val Tyr Cys Leu Lys
               900                 905                 910
   Gly Trp Asn Gln Ala Leu Ser Met Ser Asn Gly Gly Asp Asn His Trp
           915                 920                 925
   Ala Leu Phe Ala Lys Ala Val Leu Asp Arg Thr Arg Leu Ala Leu Ala
       930                 935                 940
   Ser Lys Ala Glu Trp Tyr His His Leu Leu Gln Pro Ser Ala Glu Tyr
   945                 950                 955                 960
   Leu Gly Ser Ile Leu Gly Val Asp Gln Trp Ala Leu Asn Ile Phe Thr
                   965                 970                 975
   Glu Glu Ile Ile Arg Ala Gly Ser Ala Ala Ser Leu Ser Ser Leu Leu
               980                 985                 990
   Asn Arg Leu Asp Pro Val Leu Arg Lys Thr Ala Asn Leu Gly Ser Trp
           995                 1000                1005
   Gln Ile Ile Ser Pro Val Glu Ala Val Gly Tyr Val Val Val Val Asp
       1010                1015                1020
   Glu Leu Leu Ser Val Gln Asn Glu Ile Tyr Glu Lys Pro Thr Ile Leu
   1025                1030                1035                1040
   Val Ala Lys Ser Val Lys Gly Glu Glu Glu Ile Pro Asp Gly Ala Val
                   1045                1050                1055
   Ala Leu Ile Thr Pro Asp Met Pro Asp Val Leu Ser His Val Ser Val
               1060                1065                1070
   Arg Ala Arg Asn Gly Lys Val Cys Phe Ala Thr Cys Phe Asp Pro Asn
           1075                1080                1085
   Ile Leu Ala Asp Leu Gln Ala Lys Glu Gly Arg Ile Leu Leu Leu Lys
       1090                1095                1100
   Pro Thr Pro Ser Asp Ile Ile Tyr Ser Glu Val Asn Glu Ile Glu Leu
   1105                1110                1115                1120
   Gln Ser Ser Ser Asn Leu Val Glu Ala Glu Thr Ser Ala Thr Leu Arg
                   1125                1130                1135
   Leu Val Lys Lys Gln Phe Gly Gly Cys Tyr Ala Ile Ser Ala Asp Glu
               1140                1145                1150
   Phe Thr Ser Glu Met Val Gly Ala Lys Ser Arg Asn Ile Ala Tyr Leu
           1155                1160                1165
   Lys Gly Lys Val Pro Ser Ser Val Gly Ile Pro Thr Ser Val Ala Leu
       1170                1175                1180
   Pro Phe Gly Val Phe Glu Lys Val Leu Ser Asp Asp Ile Asn Gln Gly
   1185                1190                1195                1200
   Val Ala Lys Glu Leu Gln Ile Leu Met Lys Lys Leu Ser Glu Gly Asp
                   1205                1210                1215
   Phe Ser Ala Leu Gly Glu Ile Arg Thr Thr Val Leu Asp Leu Ser Ala
               1220                1225                1230
   Pro Ala Gln Leu Val Lys Glu Leu Lys Glu Lys Met Gln Gly Ser Gly
           1235                1240                1245
   Met Pro Trp Pro Gly Asp Glu Gly Pro Lys Arg Trp Glu Gln Ala Trp
       1250                1255                1260
   Met Ala Ile Lys Lys Val Trp Ala Ser Lys Trp Asn Glu Arg Ala Tyr
   1265                1270                1275                1280
   Phe Ser Thr Arg Lys Val Lys Leu Asp His Asp Tyr Leu Cys Met Ala
                   1285                1290                1295
   Val Leu Val Gln Glu Ile Ile Asn Ala Asp Tyr Ala Phe Val Ile His
               1300                1305                1310
   Thr Thr Asn Pro Ser Ser Gly Asp Asp Ser Glu Ile Tyr Ala Glu Val
           1315                1320                1325
   Val Arg Gly Leu Gly Glu Thr Leu Val Gly Ala Tyr Pro Gly Arg Ala
       1330                1335                1340
   Leu Ser Phe Ile Cys Lys Lys Lys Asp Leu Asn Ser Pro Gln Val Leu
   1345                1350                1355                1360
   Gly Tyr Pro Ser Lys Pro Ile Gly Leu Phe Ile Lys Arg Ser Ile Ile
                   1365                1370                1375
   Phe Arg Ser Asp Ser Asn Gly Glu Asp Leu Glu Gly Tyr Ala Gly Ala
               1380                1385                1390
   Gly Leu Tyr Asp Ser Val Pro Met Asp Glu Glu Glu Lys Val Val Ile
           1395                1400                1405
   Asp Tyr Ser Ser Asp Pro Leu Ile Thr Asp Gly Asn Phe Arg Gln Thr
       1410                1415                1420
   Ile Leu Ser Asn Ile Ala Arg Ala Gly His Ala Ile Glu Glu Leu Tyr
   1425                1430                1435                1440
   Gly Ser Pro Gln Asp Ile Glu Gly Val Val Arg Asp Gly Lys Ile Tyr
                   1445                1450                1455
   Val Val Gln Thr Arg Pro Gln Met
               1460

(2) ANGABEN ZU SEQ ID NO: 3:

(i) SEQUENZKENNZEICHEN:
(A) LÄNGE: 1918 Basenpaare
(B) ART: Nucleotid
(C) STRANGFORM: Einzelstrang
(D) TOPOLOGIE: linear

(ii) ART DES MOLEKÜLS: cDNA zu mRNA

(vi) URSPRÜNLICHE HERKUNFT:
(A) ORGANISMUS: Solanum tuberosum
(B) STAMM: C.V. Desiree

(ix) MERKMAL:
(A) NAME/SCHLÜSSEL: CDS
(B) LAGE:1..1555

(xi) SEQUENZBESCHREIBUNG: SEQ ID NO: 3:

GCA GAG TGG TAC CAT CAC TTA TTG CAG CCA TCT GCC GAA TAT CTA GGA 48
Ala Glu Trp Tyr His His Leu Leu Gln Pro Ser Ala Glu Tyr Leu Gly
1               5                   10                  15

TCA ATA CTT GGG GTG GAC CAA TGG GCT TTG AAC ATA TTT ACT GAA GAA 96
Ser Ile Leu Gly Val Asp Gln Trp Ala Leu Asn Ile Phe Thr Glu Glu
            20                  25                  30

ATT ATA CGT GCT GGA TCA GCA GCT TCA TTA TCC TCT CTT CTT AAT AGA 144
Ile Ile Arg Ala Gly Ser Ala Ala Ser Leu Ser Ser Leu Leu Asn Arg
        35                  40                  45

CTC GAT CCC GTG CTT CGG AAA ACT GCA AAT CTA GGA AGT TGG CAG ATT 192
Leu Asp Pro Val Leu Arg Lys Thr Ala Asn Leu Gly Ser Trp Gln Ile
    50                  55                  60

ATC AGT CCA GTT GAA GCC GTT GGA TAT GTT GTC GTT GTG GAT GAG TTG 240
Ile Ser Pro Val Glu Ala Val Gly Tyr Val Val Val Val Asp Glu Leu
65                  70                  75                  80

CTT TCA GTT CAG AAT GAA ATC TAC GAG AAG CCC ACG ATC TTA GTA GCA 288
Leu Ser Val Gln Asn Glu Ile Tyr Glu Lys Pro Thr Ile Leu Val Ala
                85                  90                  95

AAA TCT GTT AAA GGA GAG GAG GAA ATT CCT GAT GGT GCT GTT GCC CTG 336
Lys Ser Val Lys Gly Glu Glu Glu Ile Pro Asp Gly Ala Val Ala Leu
            100                 105                 110

ATA ACA CCA GAC ATG CCA GAT GTT CTT TCA CAT GTT TCT GTT CGA GCT 384
Ile Thr Pro Asp Met Pro Asp Val Leu Ser His Val Ser Val Arg Ala
        115                 120                 125

AGA AAT GGG AAG GTT TGC TTT GCT ACA TGC TTT GAT CCC AAT ATA TTG 432
Arg Asn Gly Lys Val Cys Phe Ala Thr Cys Phe Asp Pro Asn Ile Leu
    130                 135                 140

GCT GAC CTC CAA GCA AAG GAA GGA AGG ATT TTG CTC TTA AAG CCT ACA 480
Ala Asp Leu Gln Ala Lys Glu Gly Arg Ile Leu Leu Leu Lys Pro Thr
145                 150                 155                 160

CCT TCA GAC ATA ATC TAT AGT GAG GTG AAT GAG ATT GAG CTC CAA AGT 528
Pro Ser Asp Ile Ile Tyr Ser Glu Val Asn Glu Ile Glu Leu Gln Ser
                165                 170                 175

TCA AGT AAC TTG GTA GAA GCT GAA ACT TCA GCA ACA CTT AGA TTG GTG 576
Ser Ser Asn Leu Val Glu Ala Glu Thr Ser Ala Thr Leu Arg Leu Val
            180                 185                 190

AAA AAG CAA TTT GGT GGT TGT TAC GCA ATA TCA GCA GAT GAA TTC ACA 624
Lys Lys Gln Phe Gly Gly Cys Tyr Ala Ile Ser Ala Asp Glu Phe Thr
        195                 200                 205

AGT GAA ATG GTT GGA GCT AAA TCA CGT AAT ATT GCA TAT CTG AAA GGA 672
Ser Glu Met Val Gly Ala Lys Ser Arg Asn Ile Ala Tyr Leu Lys Gly
    210                 215                 220

AAA GTG CCT TCC TCG GTG GGA ATT CCT ACG TCA GTA GCT CTT CCA TTT 720
Lys Val Pro Ser Ser Val Gly Ile Pro Thr Ser Val Ala Leu Pro Phe
225                 230                 235                 240

GGA GTC TTT GAG AAA GTA CTT TCA GAC GAC ATA AAT CAG GGA GTG GCA 768
Gly Val Phe Glu Lys Val Leu Ser Asp Asp Ile Asn Gln Gly Val Ala
                245                 250                 255

AAA GAG TTG CAA ATT CTG ACA AAA AAA CTA TCT GAA GGA GAC TTT AGC 816
Lys Glu Leu Gln Ile Leu Thr Lys Lys Leu Ser Glu Gly Asp Phe Ser
            260                 265                 270

GCT CTT GGT GAA ATT CGC ACA ACG GTT TTA GAT CTT TCG ACA CCA GCT 864
Ala Leu Gly Glu Ile Arg Thr Thr Val Leu Asp Leu Ser Thr Pro Ala
        275                 280                 285

CAA TTG GTC AAA GAG CTG AAG GAG AAG ATG CAG GGT TCT GGC ATG CCT 912
Gln Leu Val Lys Glu Leu Lys Glu Lys Met Gln Gly Ser Gly Met Pro
    290                 295                 300

TGG CCT GGT GAT GAA GGT CCA AAG CGG TGG GAA CAA GCA TGG ATG GCC 960
Trp Pro Gly Asp Glu Gly Pro Lys Arg Trp Glu Gln Ala Trp Met Ala
305                 310                 315                 320

ATA AAA AAG GTG TGG GCT TCA AAA TGG AAT GAG AGA GCA TAC TTC AGC 1008
Ile Lys Lys Val Trp Ala Ser Lys Trp Asn Glu Arg Ala Tyr Phe Ser
                325                 330                 335

ACA AGG AAG GTG AAA CTG GAT CAT GAC TAT CTG TGC ATG GCT GTC CTT 1056
Thr Arg Lys Val Lys Leu Asp His Asp Tyr Leu Cys Met Ala Val Leu
            340                 345                 350

GTT CAA GAA ATA ATA AAT GCT GAT TAT GCA TTT GTC ATT CAC ACA ACC 1104
Val Gln Glu Ile Ile Asn Ala Asp Tyr Ala Phe Val Ile His Thr Thr
        355                 360                 365

AAC CCA TCT TCC GGA GAC GAC TCA GAA ATA TAT GCC GAG GTG GTC AGG 1152
Asn Pro Ser Ser Gly Asp Asp Ser Glu Ile Tyr Ala Glu Val Val Arg
    370                 375                 380

GGC CTT GGG GAA ACA CTT GTT GGA GCT TAT CCA GGA CGT GCT TTG AGT 1200
Gly Leu Gly Glu Thr Leu Val Gly Ala Tyr Pro Gly Arg Ala Leu Ser
385                 390                 395                 400

TTT ATC TGC AAG AAA AAG GAT CTC AAC TCT CCT CAA GTG TTA GGT TAC 1248
Phe Ile Cys Lys Lys Lys Asp Leu Asn Ser Pro Gln Val Leu Gly Tyr
                405                 410                 415

CCA AGC AAA CCG ATC GGC CTT TTC ATA AAA AGA TCT ATC ATC TTC CGA 1296
Pro Ser Lys Pro Ile Gly Leu Phe Ile Lys Arg Ser Ile Ile Phe Arg
            420                 425                 430

TCT GAT TCC AAT GGG GAA GAT TTG GAA GGT TAT GCC GGT GCT GGC CTC 1344
Ser Asp Ser Asn Gly Glu Asp Leu Glu Gly Tyr Ala Gly Ala Gly Leu
        435                 440                 445

TAC GAC AGT GTA CCA ATG GAT GAG GAG GAA AAA GTT GTA ATT GAT TAC 1392
Tyr Asp Ser Val Pro Met Asp Glu Glu Glu Lys Val Val Ile Asp Tyr
    450                 455                 460

TCT TCC GAC CCA TTG ATA ACT GAT GGT AAC TTC CGC CAG ACA ATC CTG 1440
Ser Ser Asp Pro Leu Ile Thr Asp Gly Asn Phe Arg Gln Thr Ile Leu
465                 470                 475                 480

TCC AAC ATT GCT CGT GCT GGA CAT GCT ATC GAG GAG CTA TAT GGC TCT 1488
Ser Asn Ile Ala Arg Ala Gly His Ala Ile Glu Glu Leu Tyr Gly Ser
                485                 490                 495

CCT CAA GAC ATT GAG GGT GTA GTG AGG GAT GGA AAG ATT TAT GTC GTT 1536
Pro Gln Asp Ile Glu Gly Val Val Arg Asp Gly Lys Ile Tyr Val Val
            500                 505                 510

CAG ACA AGA CCA CAG ATG T GATTATATTC TCGTTGTATG TTGTTCAGAG 1585
Gln Thr Arg Pro Gln Met
        515

AAGACCACAG ATGTGATCAT ATTCTCATTG TATCAGATCT GTGACCACTT ACCTGATACC 1645

TCCCATGAAG TTACCTGTAT GATTATACGT GATCCAAAGC CATCACATCA TGTTCACCTT 1705

CAGCTATTGG AGGAGAAGTG AGAAGTAGGA ATTGCAATAT GAGGAATAAT AAGAAAAACT 1765

TTGTAAAAGC TAAATTAGCT GGGTATGATA TAGGGAGAAA TGTGTAAACA TTGTACTATA 1825

TATAGTATAT ACACACGCAT TATGTATTGC ATTATGCACT GAATAATATC GCAGCATCAA 1885

AGAAGAAATC CTTTGGGTGG TTTCAAAAAA AAA 1918

(2) ANGABEN ZU SEQ ID NO: 4:

(i) SEQUENZKENNZEICHEN:
(A) LÄNGE: 518 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 4:

Ala Glu Trp Tyr His His Leu Leu Gln Pro Ser Ala Glu Tyr Leu Gly
1               5                   10                  15

Ser Ile Leu Gly Val Asp Gln Trp Ala Leu Asn Ile Phe Thr Glu Glu
            20                  25                  30

Ile Ile Arg Ala Gly Ser Ala Ala Ser Leu Ser Ser Leu Leu Asn Arg
        35                  40                  45

Leu Asp Pro Val Leu Arg Lys Thr Ala Asn Leu Gly Ser Trp Gln Ile

50                  55                  60

Ile Ser Pro Val Glu Ala Val Gly Tyr Val Val Val Val Asp Glu Leu
65                  70                  75                  80

Leu Ser Val Gln Asn Glu Ile Tyr Glu Lys Pro Thr Ile Leu Val Ala
                85                  90                  95

Lys Ser Val Lys Gly Glu Glu Glu Ile Pro Asp Gly Ala Val Ala Leu
            100                 105                 110

Ile Thr Pro Asp Met Pro Asp Val Leu Ser His Val Ser Val Arg Ala
        115                 120                 125

Arg Asn Gly Lys Val Cys Phe Ala Thr Cys Phe Asp Pro Asn Ile Leu
    130                 135                 140

Ala Asp Leu Gln Ala Lys Glu Gly Arg Ile Leu Leu Leu Lys Pro Thr
145                 150                 155                 160

Pro Ser Asp Ile Ile Tyr Ser Glu Val Asn Glu Ile Glu Leu Gln Ser
                165                 170                 175

Ser Ser Asn Leu Val Glu Ala Glu Thr Ser Ala Thr Leu Arg Leu Val
            180                 185                 190

Lys Lys Gln Phe Gly Gly Cys Tyr Ala Ile Ser Ala Asp Glu Phe Thr
        195                 200                 205

Ser Glu Met Val Gly Ala Lys Ser Arg Asn Ile Ala Tyr Leu Lys Gly
    210                 215                 220

Lys Val Pro Ser Ser Val Gly Ile Pro Thr Ser Val Ala Leu Pro Phe
225                 230                 235                 240

Gly Val Phe Glu Lys Val Leu Ser Asp Asp Ile Asn Gln Gly Val Ala
                245                 250                 255

Lys Glu Leu Gln Ile Leu Thr Lys Lys Leu Ser Glu Gly Asp Phe Ser
            260                 265                 270

Ala Leu Gly Glu Ile Arg Thr Thr Val Leu Asp Leu Ser Thr Pro Ala
        275                 280                 285

Gln Leu Val Lys Glu Leu Lys Glu Lys Met Gln Gly Ser Gly Met Pro
    290                 295                 300

Trp Pro Gly Asp Glu Gly Pro Lys Arg Trp Glu Gln Ala Trp Met Ala
305                 310                 315                 320

Ile Lys Lys Val Trp Ala Ser Lys Trp Asn Glu Arg Ala Tyr Phe Ser
                325                 330                 335

Thr Arg Lys Val Lys Leu Asp His Asp Tyr Leu Cys Met Ala Val Leu
            340                 345                 350

Val Gln Glu Ile Ile Asn Ala Asp Tyr Ala Phe Val Ile His Thr Thr
        355                 360                 365

Asn Pro Ser Ser Gly Asp Asp Ser Glu Ile Tyr Ala Glu Val Val Arg
    370                 375                 380

Gly Leu Gly Glu Thr Leu Val Gly Ala Tyr Pro Gly Arg Ala Leu Ser
385                 390                 395                 400

Phe Ile Cys Lys Lys Lys Asp Leu Asn Ser Pro Gln Val Leu Gly Tyr
                405                 410                 415

Pro Ser Lys Pro Ile Gly Leu Phe Ile Lys Arg Ser Ile Ile Phe Arg
            420                 425                 430

Ser Asp Ser Asn Gly Glu Asp Leu Glu Gly Tyr Ala Gly Ala Gly Leu
        435                 440                 445

Tyr Asp Ser Val Pro Met Asp Glu Glu Glu Lys Val Val Ile Asp Tyr
    450                 455                 460

Ser Ser Asp Pro Leu Ile Thr Asp Gly Asn Phe Arg Gln Thr Ile Leu
465                 470                 475                 480

Ser Asn Ile Ala Arg Ala Gly His Ala Ile Glu Glu Leu Tyr Gly Ser
                485                 490                 495

Pro Gln Asp Ile Glu Gly Val Val Arg Asp Gly Lys Ile Tyr Val Val
            500                 505                 510

Gln Thr Arg Pro Gln Met
        515